# EUROPEAN PATENT SPECIFICATION

(11) **EP 2 044 058 B1**
(45) Date of publication and mention of the grant of the patent: **29.09.2010**
(21) Application number: 07766135.3
(22) Date of filing: 20.06.2007
(51) Int. Cl.: C07D 403/04, A61P 35/02, A61P 35/04, A61K 31/506

(54) **CHEMICAL COMPOUNDS**
CHEMISCHE VERBINDUNGEN
COMPOSÉS CHIMIQUES

(30) Priority: 21.06.2006 US 805353 P
(43) Date of publication of application: 08.04.2009
(73) Proprietor: AstraZeneca AB, 151 85 Södertälje (SE)
(72) Inventor: JONES, Clifford, Macclesfield Cheshire SK10 4TG (GB); MATUSIAK, Zbigniew Stanley, Macclesfield Cheshire SK10 4TG (GB)
(86) International application number: PCT/GB2007/002279
(87) International publication number: WO 2007/148070

(56) References cited:
- WO-A-2005/075461
- WO-A1-02/20512

## Description

The invention relates to pyrimidine derivatives, or pharmaceutically acceptable salts thereof, which possess cell-cycle inhibitory activity and are accordingly useful for their anti-cell-proliferation (such as anti-cancer) activity and are therefore useful in methods of treatment of the human or animal body. The invention also relates to pharmaceutical compositions containing said pyrimidine derivatives and to their use in the manufacture of a medicament.

The cell cycle is fundamental to the survival, regulation and proliferation of cells and is highly regulated to ensure that each step progresses in a timely and orderly manner. The progression of cells through the cell cycle arises from the sequential activation and de-activation of several members of the cyclin-dependent kinase (CDK) family. The activation of CDKs is dependent on their interaction with a family of intracellular proteins called cyclins. Cyclins bind to CDKs and this association is essential for CDK activity within the cell. Different cyclins are expressed and degraded at different points in the cell cycle to ensure that activation and inactivation of CDKs occurs in the correct order for progression through the cell cycle.

Moreover, CDKs appear to be downstream of a number of oncogene signalling pathways. Deregulation of CDK activity by upregulation of cyclins and/or deletion of endogenous inhibitors appears to be an important axis between mitogenic signalling pathways and proliferation of tumour cells.

Accordingly it has been recognised that an inhibitor of cell cycle kinases, particularly inhibitors of CDK1, CDK2, CDK4 and CDK6 (which operate at the G2/M, G1/S-S-G2/M and G1-S phases respectively) should be of value as an active inhibitor of cell proliferation, such as growth of mammalian cancer cells.

Tumour cells are also thought to be highly dependent on the continual transcriptional activity of RNA polymerase II to maintain appropriate levels of anti-apoptotic proteins and ensure tumour cell survival. CDK1, CDK7, CDK8 and CDK9 in particular are known to regulate the activity of RNA polymerase II through phosphorylation of the C-terminal domain of the protein. Thus, the inhibition of RNA polymerase II activity through inhibitors of these CDKs may contribute to a pro-apoptotic effect in tumour cells.

The inhibition of cell cycle kinases is expected to be of value in the treatment of disease states associated with aberrant cell cycles and cell proliferation such as cancers (solid tumours and leukemias), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

WO 02/20512, WO 03/076435, WO 03/076436, WO 03/076434, WO 03/076433 and WO 04/101549 describe certain 2-anilino-4-imidazolylpyrimidine derivatives that inhibit the effect of cell cycle kinases. The present invention is based on the discovery that a novel group of compounds inhibit the effects of CDK2, and thus possess anti-cell-proliferation properties.

Accordingly, the present invention provides a compound of formula **(I):** 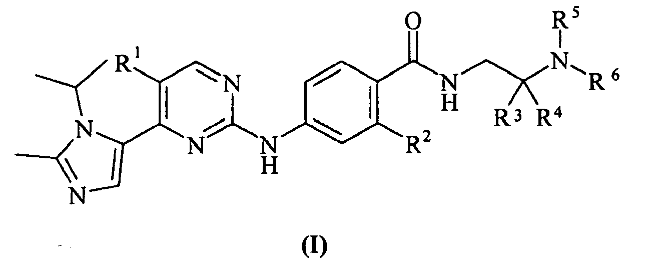 wherein:
**R¹** is hydrogen, halo or cyano;
**R²** is hydrogen or halo;
**R³, R⁴, R⁵** and R⁶ are independently selected from hydrogen and methyl optionally substituted by hydroxy;
or a pharmaceutically acceptable salt thereof.

The term "halo" refers to fluoro, chloro, bromo and iodo.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

The invention relates to any and all tautomeric forms of the compounds of the formula **(I)** that possess CDK inhibitory activity.

It is also to be understood that certain compounds of the formula (I) can exist in solvated as well as unsolvated forms such as, for example, hydrated forms. It is to be understood that the invention encompasses all such solvated forms which possess CDK inhibitory activity.

Particular values of variable groups are as follows. Such values may be used where appropriate with any of the definitions, claims or embodiments defined hereinbefore or hereinafter.

R¹ is hydrogen.

R¹ is halo.

R¹ is fluoro.

R¹ is chloro.

R¹ is hydrogen, fluoro or chloro.

R¹ is hydrogen, fluoro, chloro or cyano.

R² is hydrogen.

R² is halo.

R² is fluoro.

R² is chloro.

R² is hydrogen or fluoro.

R³ is hydrogen.

R³ is methyl.

R³ is hydrogen or methyl.

R³ is methyl optionally substituted by hydroxy.

R⁴ is hydrogen.

R⁴ is methyl.

R⁴ is methyl optionally substituted by hydroxy.

R⁴ is hydrogen or methyl.

R³ and R⁴ are hydrogen or methyl.

R⁵ is hydrogen.

R⁵ is methyl.

R⁵ is methyl optionally substituted by hydroxy.

R⁶ is hydrogen.

R⁶ is methyl.

R⁶ is methyl optionally substituted by hydroxy.

R⁶ is hydrogen or methyl.

R³ and R⁴ are both hydrogen.

R³ and R⁴ are both methyl.

R⁵ and R⁶ are both hydrogen.

R⁵ and R⁶ are both methyl.

One of R⁵ and R⁶ is methyl and the other is hydrogen or methyl.

One of R⁵ and R⁶ is hydrogen and the other is methyl.

Therefore is a further aspect of the invention there is provided a compound of formula (I) wherein:
R¹ is hydrogen, fluoro, chloro or cyano;
R² is hydrogen or fluoro;
R³ and R⁴ are hydrogen or methyl;
one of R⁵ and R⁶ is methyl and the other is hydrogen or methyl;
or a pharmaceutically acceptable salt or thereof.

Therefore is a further aspect of the invention there is provided a compound of formula (I) wherein:
R¹ is hydrogen, fluoro or chloro;
R² is hydrogen or fluoro;
R³ and R⁴ are both hydrogen or both methyl;
one of R⁵ and R⁶ is methyl and the other is hydrogen or methyl;
or a pharmaceutically acceptable salt thereof.

In another aspect of the invention, preferred compounds of the invention are any one of the Examples or a pharmaceutically acceptable salt thereof.

Preferred aspects of the invention are those which relate to the compound of formula (I) or a pharmaceutically acceptable salt thereof.

A compound of formula (I) or a pharmaceutically acceptable salt thereof may be prepared using the following process, which process (wherein variable groups are, unless otherwise specified, as defined in formula **(I))** comprises of:

### Process a) reaction of a pyrimidine of formula (II):

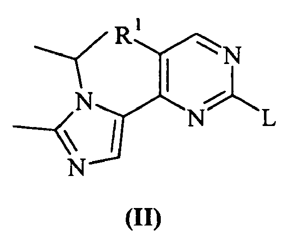

wherein L is a displaceable group; with an aniline of formula **(III):**

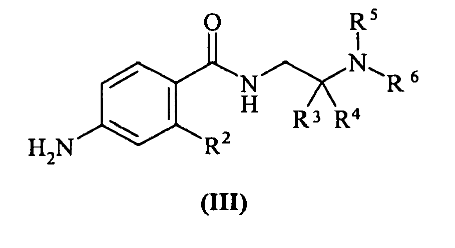

### Process b) for compounds of formula (I) wherein R¹ is not cyano; reacting a compound of formula (IV):

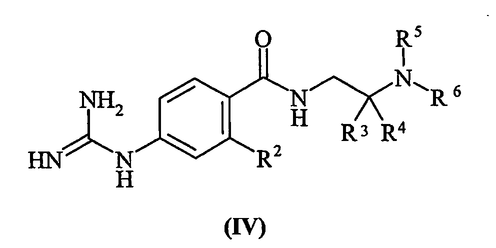

with a compound of formula **(V):** 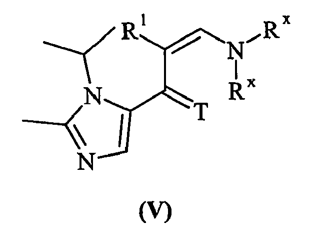 wherein T is O or S; R^{x} may be the same or different and is selected from C₁₋₆alkyl; or *Process c*) reacting an acid of formula **(VI):** 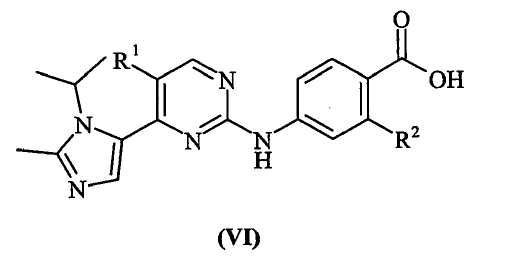 or an activated derivative thereof; with an amine of formula **(VII):** 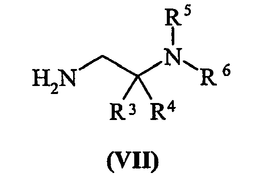 or

### Process d) reacting a pyrimidine of formula (VIII):

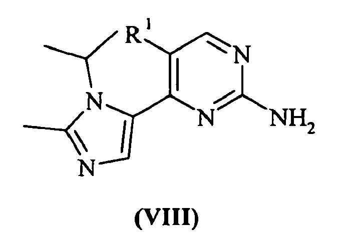

with a compound of formula **(IX):** 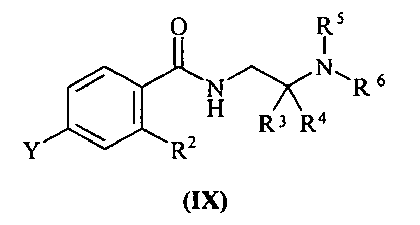 where Y is a displaceable group;
and thereafter if necessary:
i) converting a compound of the formula **(I)** into another compound of the formula **(I);**
ii) removing any protecting groups;
iii) forming a pharmaceutically acceptable salt.

L is a displaceable group, suitable values for L are for example, a halogeno or sulphonyloxy group, for example a chloro, bromo, methanesulphonyloxy or toluene-4-sulphonyloxy group.

Y is a displaceable group, suitable values for Y are for example, a halogeno or sulphonyloxy group, for example a bromo, iodo or trifluoromethanesulphonyloxy group. Preferably Y is iodo.

Specific reaction conditions for the above reactions are as follows.
*Process a*) Pyrimidines of formula **(II)** and anilines of formula **(III)** may be reacted together:
i) in the presence of a suitable solvent for example a ketone such as acetone or an alcohol such as ethanol or butanol or an aromatic hydrocarbon such as toluene or *N*-methyl pyrrolidine, optionally in the presence of a suitable acid for example an inorganic acid such as hydrochloric acid or sulphuric acid, or an organic acid such as acetic acid or formic acid (or a suitable Lewis acid) and at a temperature in the range of 0°C to reflux, preferably reflux; or
ii) under standard Buchwald conditions (for example see J. Am. Chem. Soc., 118, 7215; J. Am. Chem. Soc., 119, 8451; J. Org. Chem., 62, 1568 and 6066) for example in the presence of palladium acetate, in a suitable solvent for example an aromatic solvent such as toluene, benzene or xylene, with a suitable base for example an inorganic base such as caesium carbonate or an organic base such as potassium-*t*-butoxide, in the presence of a suitable ligand such as 2,2'-bis(diphenylphosphino)-1,1'-binaphthyl and at a temperature in the range of 25 to 80°C.

Pyrimidines of the formula **(II)** where L is chloro may be prepared according to *Scheme 1:*

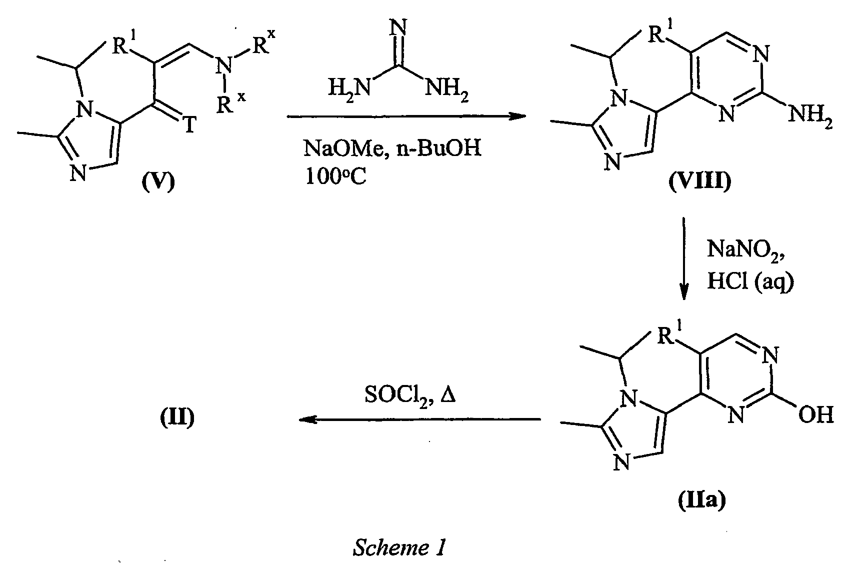

Anilines of formula **(III)** are commercially available compounds, or they are known in the literature, or they are prepared by standard processes known in the art.
*Process b*) Compounds of formula **(IV)** and compounds of formula **(V)** are reacted together in a suitable solvent such as *N*-methylpyrrolidinone or butanol at a temperature in the range of 100-200°C, preferably in the range of 150-170°C. The reaction is preferably conducted in the presence of a suitable base such as, for example, sodium hydride, sodium methoxide or potassium carbonate.

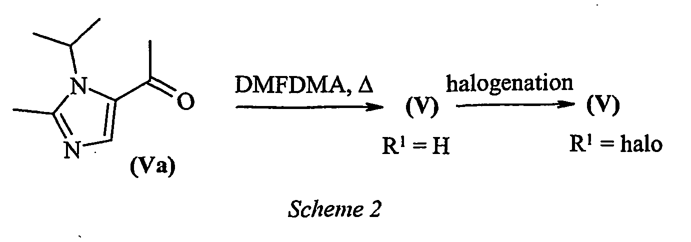

1-(1-Isopropyl-2-methyl-1*H*-imidazol-5-yl)ethanone **(Va)** is Method 3 in WO03/076436.

Compounds of formula **(IV)** are commercially available compounds, or they are known in the literature, or they are prepared by standard processes known in the art.
*Process c*) Acids and amines may be coupled together in the presence of a suitable coupling reagent. Standard peptide coupling reagents known in the art can be employed as suitable coupling reagents, or for example carbonyldiimidazole and dicyclohexyl-carbodiimide, optionally in the presence of a catalyst such as dimethylaminopyridine or 4-pyrrolidinopyridine, optionally in the presence of a base for Example triethylamine, pyridine, or 2,6-di-*alkyl*-pyridines such as 2,6-lutidine or 2,6-di-*tert*-butylpyridine. Suitable solvents include dimethylacetamide, dichloromethane, benzene, tetrahydrofuran and dimethylformamide. The coupling reaction may conveniently be performed at a temperature in the range of -40 to 40°C.

Suitable activated acid derivatives include acid halides, for example acid chlorides, and active esters, for example pentafluorophenyl esters. The reaction of these types of compounds with amines is well known in the art, for example they may be reacted in the presence of a base, such as those described above, and in a suitable solvent, such as those described above. The reaction may conveniently be performed at a temperature in the range of -40 to 40°C.

Compounds of formula **(VI)** may be prepared by adapting *Process a*) or *b*).

Amines of formula **(VII)** are commercially available compounds, or they are known in the literature, or they are prepared by standard processes known in the art.
*Process d*) Compounds of formula **(VIII)** and amines of formula **(IX)** may be reacted together under standard Buchwald conditions as described in *Process a.*

The synthesis of compounds of formula **(VIII)** is described in *Scheme 1.*

Compounds of formula **(IX)** are commercially available compounds, or they are known in the literature, or they are prepared by standard processes known in the art.

It will be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t*-butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

As stated hereinbefore the compounds defined in the present invention possesses anti-cell-proliferation activity such as anti-cancer activity which is believed to arise from the CDK inhibitory activity of the compound. These properties may be assessed, for example, using the procedure set out below:-

### Assay

The following abbreviations have been used :-
HEPES is *N*-[2-Hydroxyethyl]piperazine-*N*'-[2-ethanesulfonic acid]
DTT is Dithiothreitol
PMSF is Phenylmethylsulphonyl fluoride

The compounds were tested in an *in vitro* kinase assay in 96 well format using Scintillation Proximity Assay (SPA - obtained from Amersham) for measuring incorporation of [γ-33-P]-Adenosine Triphosphate into a test substrate (GST-Retinoblastoma protein; GST-Rb). In each well was placed the compound to be tested (diluted in DMSO and water to correct concentrations) and in control wells either roscovitine as an inhibitor control or DMSO as a positive control.

Approximately 0.2µl of CDK2/Cyclin E partially-purified enzyme (amount dependent on enzyme activity) diluted in 25µl incubation buffer was added to each well then 20µl of GST-Rb/ATP/ATP33 mixture (containing 0.5µg GST-Rb and 0.2µM ATP and 0.14µCi [y-33-P]-Adenosine Triphosphate in incubation buffer), and the resulting mixture shaken gently, then incubated at room temperature for 60 minutes.

To each well was then added 150µL stop solution containing (0.8 mg/well of Protein A-PVT SPA bead (Amersham)), 20µM/well of Anti-Glutathione Transferase, Rabbit IgG (obtained from Molecular Probes), 61 mM EDTA and 50 mM HEPES pH 7.5 containing 0.05% sodium azide.

The plates were sealed with Topseal-S plate sealers, left for two hours then spun at 2500rpm, 1124xg., for 5 minutes. The plates were read on a Topcount for 30 seconds per well.

The incubation buffer used to dilute the enzyme and substrate mixes contained 50 mM HEPES pH7.5, 10 mM MnCl₂, 1 mM DTT, 100µM Sodium vanadate, 100µM NaF, 10 mM Sodium Glycerophosphate, BSA (1 mg/ml final).

### Test substrate

In this assay only part of the retinoblastoma protein (Science 1987 Mar13;235(4794):1394-1399; Lee W.H., Bookstein R., Hong F., Young L.J., Shew J.Y., Lee E.Y.) was used, fused to a GST tag. PCR of retinoblastoma gene encoding amino acids 379-928 (obtained from retinoblastoma plasmid ATCC pLRbRNL) was performed, and the sequence cloned into pGEx 2T fusion vector (Smith D.B. and Johnson, K.S. Gene 67, 31 (1988); which contained a tac promoter for inducible expression, internal lac I^{q} gene for use in any E.Coli host, and a coding region for thrombin cleavage - obtained from Pharmacia Biotech) which was used to amplify amino acids 792-928. This sequence was again cloned into pGEx 2T.

The retinoblastoma 792-928 sequence so obtained was expressed in E.Coli (BL21 (DE3) pLysS cells) using standard inducible expression techniques, and purified as follows.

E.coli paste was resuspended in 10 ml/g of NETN buffer (50 mM Tris pH 7.5, 120 mM NaCl, 1 mM EDTA, 0.5%v/v NP-40, 1 mM PMSF, 1 µg/ml leupeptin, 1 µg/ml aprotinin and 1 µg/ml pepstatin) and sonicated for 2 x 45 seconds per 100 ml homogenate. After centrifugation, the supernatant was loaded onto a 10 ml glutathione Sepharose column (Pharmacia Biotech, Herts, UK), and washed with NETN buffer. After washing with kinase buffer (50 mM HEPES pH 7.5, 10 mM MgCl₂, 1 mM DTT, 1 mM PMSF, 1 µg/ml leupeptin, 1 µg/ml aprotinin and 1 µg/ml pepstatin) the protein was eluted with 50 mM reduced glutathione in kinase buffer. Fractions containing GST-Rb(792-927) were pooled and dialysed overnight against kinase buffer. The final product was analysed by Sodium Dodeca Sulfate (SDS) PAGE (Polyacrylamide gel) using 8-16% Tris-Glycine gels (Novex, San Diego, USA).

### CDK2 and Cyclin E

The open reading frames of CDK2 and Cyclin E were isolated by reverse transcriptase-PCR using HeLa cell and activated T cell mRNA as a template and cloned into the insect expression vector pVL1393 (obtained from Invitrogen 1995 catalogue number: V1392-20). CDK2 and cyclin E were then dually expressed [using a standard virus Baculogold co-infection technique] in the insect SF21 cell system (Spodoptera Frugiperda cells derived from ovarian tissue of the Fall Army Worm - commercially available).

### Example production of Cyclin E/CDK2

The following Example provides details of the production of Cyclin E/CDK2 in SF21 cells (in TC100 + 10% FBS(TCS) + 0.2% Pluronic) having dual infection MOI 3 for each virus of Cyclin E & CDK2.

SF21 cells grown in a roller bottle culture to 2.33 x 10⁶ cells/ml were used to inoculate 10 x 500 ml roller bottles at 0.2 x 10E6 cells/ml. The roller bottles were incubated on a roller rig at 28°C.

After 3 days (72 hrs.) the cells were counted, and the average from 2 bottles found to be 1.86 x 10E6 cells/ml. (99% viable). The cultures were then infected with the dual viruses at an MOI 3 for each virus.

The viruses were mixed together before addition to the cultures, and the cultures returned to the roller rig 28°C.

After 2 days (48 hrs.) post infection the 5 Litres of culture was harvested. The total cell count at harvest was 1.58 x 10E6 cells/ml.(99% viable). The cells were spun out at 2500rpm, 30 mins., 4°C in Heraeus Omnifuge 2.0 RS in 250 ml. lots. The supernatant was discarded.

### Partial co-purification of CDK2 and Cyclin E

Sf21 cells were resuspended in lysis buffer (50 mM Tris pH 8.2, 10 mM MgCl₂, 1 mM DTT, 10 mM glycerophosphate, 0.1 mM sodium orthovanadate, 0.1 mM NaF, 1 mM PMSF, 1 µg/ml leupeptin and 1 µg/ml aprotinin) and homogenised for 2 minutes in a 10 ml Dounce homgeniser. After centrifugation, the supernatant was loaded onto a Poros HQ/M 1.4/100 anion exchange column (PE Biosystems, Hertford, UK). CDK2 and Cyclin E were coeluted at the beginning of a 0-1M NaCl gradient (run in lysis buffer minus protease inhibitors) over 20 column volumes. Co-elution was checked by western blot using both anti-CDK2 and anti-Cyclin E antibodies (Santa Cruz Biotechnology, California, US).

By analogy, assays designed to assess inhibition of CDK1 and CDK4 may be constructed. CDK2 (EMBL Accession No. X62071) may be used together with Cyclin A or Cyclin E (see EMBL Accession No. M73812), and further details for such assays are contained in PCT International Publication No. WO99/21845, the relevant Biochemical & Biological Evaluation sections of which are hereby incorporated by reference.

Although the pharmacological properties of the compounds of the formula **(I)** vary with structural change, in general activity possessed by compounds of the formula **(I)** may be demonstrated at IC₅₀ concentrations or doses in the range 250µM to 1nM.

When tested in the above in-vitro assay the CDK2 inhibitory activity of Example 2 was measured as IC₅₀ = 3 nM.

According to a further aspect of the invention there is provided a pharmaceutical composition which comprises a pyrimidine derivative of the formula **(I),** or a pharmaceutically acceptable salt thereof, as defined hereinbefore in association with a pharmaceutically-acceptable diluent or carrier.

The composition may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository.

In general the above compositions may be prepared in a conventional manner using conventional excipients.

The compound of formula **(I)** will normally be administered to a warm-blooded animal at a unit dose within the range 5-5000 mg per square meter body area of the animal, i.e. approximately 0.1-80 mg/kg, and this normally provides a therapeutically-effective dose. A unit dose form such as a tablet or capsule will usually contain, for example 1-250 mg of active ingredient. Preferably a daily dose in the range of 1-50 mg/kg is employed. However the daily dose will necessarily be varied depending upon the host treated, the particular route of administration, and the severity of the illness being treated. Accordingly the optimum dosage may be determined by the practitioner who is treating any particular patient.

We have found that the compounds defined in the present invention, or a pharmaceutically acceptable salt thereof, are effective cell cycle inhibitors (anti-cell proliferation agents), which property is believed to arise from their CDK inhibitory properties. Accordingly the compounds of the present invention are expected to be useful in the treatment of diseases or medical conditions mediated alone or in part by CDK enzymes, i.e. the compounds may be used to produce a CDK inhibitory effect in a warm-blooded animal in need of such treatment. Thus the compounds of the present invention provide a method for treating the proliferation of malignant cells characterised by inhibition of CDK enzymes, i.e. the compounds may be used to produce an anti-proliferative and potentially apoptotic effect mediated alone or in part by the inhibition of CDKs. Particularly, an inhibitory effect is produced by preventing entry into or progression through the S phase by inhibition of CDK2, CDK4 and/or CDK6, especially CDK2 and entry into or progression through M phase by inhibition of CDK 1. Apoptotic effects may also be envisaged through down-regulation of RNA polymerase II activity by inhibition of CDK1, CDK7, CDK8 and in particular, CDK9. Such a compound of the invention is expected to possess a wide range of anti-cancer properties as CDKs have been implicated in many common human cancers such as leukaemia and breast, lung, colon, rectal, stomach, prostate, bladder, pancreas and ovarian cancer. Thus it is expected that a compound of the invention will possess anti-cancer activity against these cancers. It is in addition expected that a compound of the present invention will possess activity against a range of leukaemias, lymphoid malignancies and solid tumours such as carcinomas and sarcomas in tissues such as the liver, kidney, prostate and pancreas. In particular such compounds of the invention are expected to slow advantageously the growth of primary and recurrent solid tumours of, for example, the colon, breast, prostate, lungs and skin. More particularly such compounds of the invention, or a pharmaceutically acceptable salt thereof, are expected to inhibit the growth of those primary and recurrent solid tumours which are associated with CDKs, especially those tumours which are significantly dependent on CDKs for their growth and spread, including for example, certain tumours of the colon, breast, prostate, lung, vulva and skin.

It is further expected that a compound of the present invention will possess activity against other cell-proliferation diseases in a wide range of other disease states including leukaemias, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

Thus according to this aspect of the invention there is provided a compound of the formula (I), or a pharmaceutically acceptable salt thereof, as defined hereinbefore for use as a medicament.

In one aspect of the invention, where a cell cycle inhibitory effect is referred to this refers to inhibition of CDK1. In a further aspect of the invention, this refers to inhibition of CDK2. In a further aspect of the invention, this refers to inhibition of CDK4. In a further aspect of the invention, this refers to inhibition of CDK5. In a further aspect of the invention, this refers to inhibition of CDK6. In a further aspect of the invention, this refers to inhibition of CDK7. In a further aspect of the invention, this refers to inhibition of CDK8. In a further aspect of the invention, this refers to inhibition of CDK9.

In a further aspect of the invention there is provided the use of a compound of the formula **(I),** or a pharmaceutically acceptable salt thereof, as defined hereinbefore in the manufacture of a medicament for the treatment of cancer.

In a further aspect of the invention there is provided the use of a compound of the formula **(I),** or a pharmaceutically acceptable salt thereof, as defined hereinbefore in the manufacture of a medicament for the treatment of leukaemia or lymphoid malignancies or cancer of the breast, lung, colon, rectum, stomach, liver, kidney, prostate, bladder, pancreas, vulva, skin or ovary.

According to a further feature of the invention, there is provided the use of a compound of the formula **(I),** or a pharmaceutically acceptable salt thereof, as defined herein before in the manufacture of a medicament for the treatment of cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

In a further aspect of the invention there is provided a pharmaceutical composition which comprises a compound of the formula **(I),** or a pharmaceutically acceptable salt thereof, as defined herein before and a pharmaceutically-acceptable diluent or carrier.

In a further aspect of the invention there is provided a pharmaceutical composition which comprises a compound of the formula **(I),** or a pharmaceutically acceptable salt thereof, as defined herein before and a pharmaceutically-acceptable diluent or carrier for use as a medicament.

In a further aspect of the invention there is provided a pharmaceutical composition which comprises a compound of the formula **(I),** or a pharmaceutically acceptable salt thereof, as defined herein before and a pharmaceutically-acceptable diluent or carrier for use in the treatment of cancer.

In a further aspect of the invention there is provided a pharmaceutical composition which comprises a compound of the formula **(I),** or a pharmaceutically acceptable salt thereof, as defined herein before and a pharmaceutically-acceptable diluent or carrier for use in the treatment of leukaemia or lymphoid malignancies or cancer of the breast, lung, colon, rectum, stomach, liver, kidney, prostate, bladder, pancreas, vulva, skin or ovary.

In a further aspect of the invention there is provided a pharmaceutical composition which comprises a compound of the formula **(I),** or a pharmaceutically acceptable salt thereof, as defined herein before and a pharmaceutically-acceptable diluent or carrier for use in the treatment of cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

In a further aspect of the invention there is provided the use of a compound of the formula **(I),** or a pharmaceutically acceptable salt thereof, as defined hereinbefore, in the treatment of cancer.

In a further aspect of the invention there is provided the use of a compound of the formula **(I),** or a pharmaceutically acceptable salt thereof, as defined hereinbefore in the treatment of leukaemia or lymphoid malignancies or cancer of the breast, lung, colon, rectum, stomach, liver, kidney, prostate, bladder, pancreas, vulva, skin or ovary.

According to a further feature of the invention, there is provided the use of a compound of the formula **(I),** or a pharmaceutically acceptable salt thereof, as defined herein before in the treatment of cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

Preventing cells from entering DNA synthesis by inhibition of essential S-phase initiating activities such as CDK2 initiation may also be useful in protecting normal cells of the body from toxicity of cycle-specific pharmaceutical agents. Inhibition of CDK2 or 4 will prevent progression into the cell cycle in normal cells which could limit the toxicity of cycle-specific pharmaceutical agents which act in S-phase, G2 or mitosis. Such protection may result in the prevention of hair loss normally associated with these agents.

A compound of formula **(I)** as defined above or a pharmaceutically acceptable salt thereof may be used as a cell protective agent.

A compound of formula **(I)** as defined above or a pharmaceutically acceptable salt thereof may be used in preventing hair loss arising from the treatment of malignant conditions with pharmaceutical agents.

Examples of pharmaceutical agents for treating malignant conditions that are known to cause hair loss include alkylating agents such as ifosfamide and cyclophosphamide; antimetabolites such as methotrexate, 5-fluorouracil, gemcitabine and cytarabine; vinca alkaloids and analogues such as vincristine, vinbalstine, vindesine, vinorelbine; taxanes such as paclitaxel and docetaxel; topoisomerase I inhibitors such as irintotecan and topotecan; cytotoxic antibiotics such as doxorubicin, daunorubicin, mitoxantrone, actinomycin-D and mitomycin; and others such as etoposide and tretinoin.

The compound of formula **(I),** or a pharmaceutically acceptable salt thereof, may be administered in association with a one or more of the above pharmaceutical agents. In this instance the compound of formula **(I)** may be administered by systemic or non systemic means. Particularly the compound of formula **(I)** my may administered by non-systemic means, for example topical administration.

Therefore we provide a method of preventing hair loss during treatment for one or more malignant conditions with pharmaceutical agents, in a warm-blooded animal, such as man, which comprises administering to said animal an effective amount of a compound of formula **(I),** or a pharmaceutically acceptable thereof.

We provide a method of preventing hair loss during treatment for one or more malignant conditions with pharmaceutical agents, in a warm-blooded animal, such as man, which comprises administering to said animal an effective amount of a compound of formula **(I),** or a pharmaceutically acceptable thereof in simultaneous, sequential or separate administration with an effective amount of said pharmaceutical agent.

The compound of formula **(I)** may be provided in a kit comprising:
a) a compound of formula **(I),** or a pharmaceutically acceptable salt thereof, in a first unit dosage form;
b) a pharmaceutical agent for treating malignant conditions that is known to cause hair loss; in a second unit dosage form; and
c) container means for containing said first and second dosage forms.

A compound of the formula **(I),** or a pharmaceutically acceptable salt thereof, may be used in the manufacture of a medicament for the prevention of hair loss during treatment of malignant conditions with pharmaceutical agents.

A compound of formula **(I)** may be used in a combination treatment for the prevention of hair loss comprising the administration of an effective amount of a compound of the formula **(I),** or a pharmaceutically acceptable salt or thereof, optionally together with a pharmaceutically acceptable diluent or carrier, with the simultaneous, sequential or separate administration of an effective amount of a pharmaceutical agent for treatment of malignant conditions to a warm-blooded animal, such as man.

As stated above the size of the dose required for the therapeutic or prophylactic treatment of a particular cell-proliferation disease will necessarily be varied depending on the host treated, the route of administration and the severity of the illness being treated. A unit dose in the range, for example, 1-80 mg/kg, preferably 1-50 mg/kg is envisaged.

The CDK inhibitory activity defined hereinbefore may be applied as a sole therapy or may involve, in addition to a compound of the invention, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the cell cycle inhibitory treatment defined hereinbefore may be: surgery, radiotherapy or chemotherapy. Such chemotherapy may cover three main categories of therapeutic agent:
(i) other cell cycle inhibitory agents that work by the same or different mechanisms from those defined hereinbefore;
(ii) cytostatic agents such as antioestrogens (for example tamoxifen,toremifene, raloxifene, droloxifene, iodoxyfene), progestogens (for example megestrol acetate), aromatase inhibitors (for example anastrozole, letrazole, vorazole, exemestane), antiprogestogens, antiandrogens (for example flutamide, nilutamide, bicalutamide, cyproterone acetate), LHRH agonists and antagonists (for example goserelin acetate, luprolide), inhibitors of testosterone 5α-dihydroreductase (for example finasteride), anti-invasion agents (for example metalloproteinase inhibitors like marimastat and inhibitors of urokinase plasminogen activator receptor function) and inhibitors of growth factor function, (such growth factors include for example platelet derived growth factor and hepatocyte growth factor such inhibitors include growth factor antibodies, growth factor receptor antibodies, tyrosine kinase inhibitors and serine/threonine kinase inhibitors); and
(iii) antiproliferative/antineoplastic drugs and combinations thereof, as used in medical oncology, such as antimetabolites (for example antifolates like methotrexate, fluoropyrimidines like 5-fluorouracil, purine and adenosine analogues, cytosine arabinoside); antitumour antibiotics (for example anthracyclines like doxorubicin, daunomycin, epirubicin and idarubicin, mitomycin-C, dactinomycin, mithramycin); platinum derivatives (for example cisplatin, carboplatin); alkylating agents (for example nitrogen mustard, melphalan, chlorambucil, busulphan, cyclophosphamide, ifosfamide, nitrosoureas, thiotepa); antimitotic agents (for example vinca alkaloids like vincristine and taxoids like taxol, taxotere); topoisomerase inhibitors (for example epipodophyllotoxins like etoposide and teniposide, amsacrine, topotecan). According to this aspect of the invention there is provided a pharmaceutical product comprising a compound of the formula **(I)** as defined hereinbefore and an additional anti-tumour substance as defined hereinbefore for the conjoint treatment of cancer.

In addition to their use in therapeutic medicine, the compounds of formula **(I)** and their pharmaceutically acceptable salts are also useful as pharmacological tools in the development and standardisation of in vitro and *in vivo* test systems for the evaluation of the effects of inhibitors of cell cycle activity in laboratory animals such as cats, dogs, rabbits, monkeys, rats and mice, as part of the search for new therapeutic agents.

In the above other pharmaceutical composition, process, method, use and medicament manufacture features, the alternative and preferred embodiments of the compounds of the invention described herein also apply.

### Examples

The invention will now be illustrated by the following non limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulphate; evaporation of solvent was carried out using a rotary evaporator under reduced pressure (600-4000 Pascals; 4.5-30 mmHg) with a bath temperature of up to 60°C;
(iii) chromatography means flash chromatography on silica gel; thin layer chromatography (TLC) was carried out on silica gel plates;
(iv) in general, the course of reactions was followed by TLC and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-d₆) as solvent unless otherwise indicated;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺;
(xi) unless stated otherwise compounds containing an asymmetrically substituted carbon and/or sulphur atom have not been resolved;
(xii) where a synthesis is described as being analogous to that described in a previous example the amounts used are the millimolar ratio equivalents to those used in the previous example;
(xiii) the following abbreviations have been used:
   - THF: tetrahydrofuran;
   - DMF: *N,N*-dimethylformamide;
   - EtOAc: ethyl acetate;
   - MeOH: methanol;
   - ether: diethyl ether;
   - EtOH: ethanol;
   - DCM: dichloromethane;
   - DMSO: dimethylsulphoxide;
   - DIAD: diisopropyl azodicarboxylate;
   - TEA: triethylamine;
   - HBTU: *O*-benzotriazol-1-yl-*N,N,N',N'*-tetramethyluronium hexafluorophosphate;
   - DIPEA: *N,N*-diisopropylethylamine;
   - MPLC: medium pressure liquid chromatography;
   - RPHPLC: reverse phase high performance liquid chromatography; and
   - Xantphos: 9,9-dimethyl-4,5-bis(diphenylphosphino)xanthene;
(xiv) where an SCX-2 column is referred to, this means an "ion exchange" extraction cartridge for adsorption of basic compounds, i.e. a polypropylene tube containing a benzenesulphonic acid based strong cation exchange sorbent, used according to the manufacturers instructions obtained from International Sorbent Technologies Limited, Dyffryn Business Park, Hengeod, Mid Glamorgan, UK, CF82 7RJ.

### Example 1

### N-(2-Dimethylaminoethyl)-4-[[4-(2-methyl-3-propan-2-yl-imidazol-4-yl)pyrimidin-2-yl]amino]benzamide

4-(3-Isopropyl-2-methyl-3*H*-imidazol-4-yl)-pyrimidin-2-ylamine (Method 1; 0.15 g), palladium acetate (10 mg), Xantphos (48 mg), caesium carbonate (0.45 g) and N-(2-dimethylaminoethyl)-4-iodo-benzamide (Method 4; 0.24 g) were added to dioxane (9 ml) under an inert atmosphere and heated at reflux for 9 hrs. The reaction mixture was then concentrated *in vacuo,* the residue loaded onto a SC-X-2 column in MeOH, washed with MeOH then eluted with 7N NH₃ in MeOH. Further purification was achieved using RPHPLC to give the title compound (0.212 g); NMR (400.132 MHz, CDCl₃) 8.38 (d, 1H), 7.80 (d, 2H), 7.68 (d, 2H), 7.38 (s, 1H), 7.22 (s, 1H), 6.96 (d, 1H), 6.83 (brs, 1H), 5.65 (septet, 1H), 3.54 (q, 2H), 2.60 (s, 3H), 2.57 (t, 2H), 2.31 (s, 6H), 1.55 (d, 6H); m/z 408.

### Example 2

### N-(2-Dimethylaminoethyl)-2-fluoro-4-[[4-(2-methyl-3-propan-2-yl-imidazol-4-yl)pyrimidin-2-yl]amino]benzamide

The title compound was prepared by the procedure of Example 1 and on the same scale, using 4-bromo-N-(2-dimethylaminoethyl)-2-fluoro-benzamide (Method 5) and 4-(3-isopropyl-2-methyl-3*H*-imidazol-4-yl)-pyrimidin-2-ylamine (Method 1); NMR (400.132 MHz, CDCl₃) 8.40 (d, 1H), 8.04 (t, 1H), 7.80 (dd, 1H), 7.53 (s, 1H), 7.40 (s, 1H), 7.24 (dd, 1H), 7.00 (d, 1H), 5.64 (septet, 1H), 3.56 (q, 2H), 2.60 (s, 3H), 2.53 (t, 3H), 2.29 (s, 6H), 1.56 (d, 6H); m/z 426.

### Example 3

### N-(2-Dimethylaminoethyl)-4-[[5-fluoro-4-(2-methyl-3-propan-2-yl-imidazol-4-yl)pyrimidin-2-yl]amino]benzamide

The title compound was prepared by the procedure of Example 1 and on the same scale, using N-(2-dimethylaminoethyl)-4-iodo-benzamide (Method 4) and 5-fluoro-4-(3-isopropyl-2-methyl-3*H*-imidazol-4-yl)-pyrimidin-2-ylamine (Method 3). NMR (400.132 MHz, CDCl₃) 8.31 (d, 1H), 7.78 (d, 2H), 7.63 (d, 2H), 7.59 (d, 1H), 7.22 (s, 1H), 6.78 - 6.72 (brs, 1H), 5.57 (septet, 1H), 3.52 (q, 2H), 2.62 (s, 3H), 2.52 (t, 2H), 2.28 (s, 6H), 1.54 (d, 6H); m/z 426.

### Example 4

### N-(2-Dimethylaminoethyl)-2-fluoro-4-[[5-fluoro-4-(2-methyl-3-propan-2-yl-imidazol-4-yl)pyrimidin-2-yl]amino]benzamide

The title compound was prepared by the procedure of Example 1 and on the same scale, using 4-bromo-N-(2-dimethylaminoethyl)-2-fluoro-benzamide (Method 5) and 5-fluoro-4-(3-isopropyl-2-methyl-3H-imidazol-4-yl)-pyrimidin-2-ylamine (Method 3). NMR (400.132 MHz, CDCl₃) 8.33 (d, 1H), 8.04 (t, 1H), 7.73 (dd, 1H), 7.61 (d, 1H), 7.34 (s, 1H), 7.21 - 7.15 (m, 2H), 5.55 (septet, 1H), 3.56 (q, 2H), 2.63 (s, 3H), 2.52 (t, 2H), 2.28 (s, 6H), 1.56 (d, 6H); m/z 444.

### Example 5

### 4-[[5-Chloro-4-(2-methyl-3-propan-2-yl-imidazol-4-yl)pyrimidin-2-yl]amino]-N-(2-dimethylaminoethyl)-2-fluoro-benzamide

The title compound was prepared by the procedure of Example 1 and on the same scale, using 4-bromo-N-(2-dimethylaminoethyl)-2-fluoro-benzamide (Method 5) and 5-chloro-4-(2-methyl-3-propan-2-yl-imidazol-4-yl)pyrimidin-2-amine (Method 5 in WO05/075461). NMR (400.132 MHz, CDCl₃) 8.48 (s, 1H), 8.03 (t, 1H), 7.77 (dd, 1H), 7.68 (s, 1H), 7.53 (s, 1H), 7.24 - 7.19 (m, 2H), 4.95 (septet, 1H), 3.56 (q, 2H), 2.61 (s, 3H), 2.54 (t, 2H), 2.29 (s, 6H), 1.50 (d, 6H); m/z 460.

### Example 6

### N-(2-Methylaminoethyl)-4-[[4-(2-methyl-3-propan-2-yl-imidazol-4-yl)pyrimidin-2-yl]amino]benzamide

The title compound was prepared by the procedure of Example 1 and on the same scale, using 4-iodo-N-(2-methylaminoethyl)benzamide (Method 6) and 4-(3-isopropyl-2-methyl-3*H*-imidazol-4-yl)-pyrimidin-2-ylamine (Method 1). NMR (400.132 MHz, CDCl₃) 8.36 (d, 1H), 7.81 (d, 2H), 7.66 (d, 2H), 7.41 (s, 1H), 7.37 (s, 1H), 7.00 (t, 1H), 6.95 (d, 1H), 5.64 (septet, 1H), 3.59 (q, 2H), 2.90 (t, 2H), 2.58 (s, 3H), 2.50 (s, 3H), 1.53 (d, 6H); m/z 394.

### Example 7

### 4-[[5-Fluoro-4-(2-methyl-3-propan-2-yl-imidazol-4-yl)pyrimidin-2-yl]amino]-N-(2-methylaminoethyl)benzamide

The title compound was prepared by the procedure of Example 1 and on the same scale, using 4-iodo-N-(2-methylaminoethyl)benzamide (Method 6) and 5-fluoro-4-(3-isopropyl-2-methyl-3H-imidazol-4-yl)-pyrimidin-2-ylamine (Method 3). NMR (400.132 MHz, CDCl₃) 8.30 (d, 1H), 7.79 (d, 2H), 7.62 (d, 2H), 7.59 (d, 1H), 7.34 (s, 1H), 6.79 (s, 1H), 5.57 (septet, 1H), 3.55 (q, 2H), 2.84 (t, 2H), 2.61 (s, 3H), 2.46 (s, 3H), 1.83 - 1.68 (m, 1H), 1.53 (d, 6H); m/z 412.

### Example 8

### 4-[[5-Chloro-4-(2-methyl-3-nropan-2-yl-imidazol-4-yl)pyrimidin-2-yl]amino]-N-(2-methylaminoethyl)benzamide

The title compound was prepared by the procedure of Example 1 and on the same scale, using 4-iodo-N-(2-methylaminoethyl)benzamide (Method 6) and 5-chloro-4-(2-methyl-3-propan-2-yl-imidazol-4-yl)pyrimidin-2-amine (Method 5 in WO05/075461). NMR (400.132 MHz, CDCl₃) 8.45 (s, 1H), 7.78 (d, 2H), 7.63 (d, 2H), 7.52 (s, 1H), 7.44 (s, 1H), 6.80 (s, 1H), 4.95 (septet, 1H), 3.55 (q, 2H), 2.84 (t, 2H), 2.59 (s, 3H), 2.46 (s, 3H), 1.48 (d, 6H); m/z 428.

### Example 9

### 4-[[5-Chloro-4-(2-methyl-3-propan-2-yl-imidazol-4-yl)pyrimidin-2-yl]amino]-2-fluoro-N-(2-methylaminoethyl)benzamide

The title compound was prepared by the procedure of Example 1 and on the same scale, using 4-bromo-2-fluoro-N-(2-methylaminoethyl)benzamide (Method 7) and 5-chloro-4-(2-methyl-3-propan-2-yl-imidazol-4-yl)pyrimidin-2-amine (Method 5 in WO05/075461). NMR (400.132 MHz, CDCl₃) 8.49 (s, 1H), 8.05 (t, 1H), 7.78 (dd, 1H), 7.54 (s, 1H), 7.41 (s, 1H), 7.19 (dd, 1H), 7.12 - 7.09 (m, 1H), 4.96 (septet, 1H), 3.58 (q, 2H), 2.84 (t, 2H), 2.61 (s, 3H), 2.47 (s, 3H), 1.51 (d, 6H); m/z 446.

### Example 10

### 2-Fluoro-N-(2-methylaminoethyl)-4-[[4-(2-methyl-3-propan-2-yl-imidazol-4-yl)pyrimidin-2-yl]amino]benzamide

The title compound was prepared by the procedure of Example 1 and on the same scale, using 4-bromo-2-fluoro-N-(2-methylaminoethyl)benzamide (Method 7) and 4-(3-isopropyl-2-methyl-3*H*-imidazol-4-yl)-pyrimidin-2-ylamine (Method 1). NMR (400.132 MHz, CDCl₃) 8.40 (d, 1H), 8.03 (t, 1H), 7.91 (s, 1H), 7.81 (d, 1H), 7.39 (s, 1H), 7.24 (d, 1H), 7.14 - 7.11 (m, 1H), 7.00 (d, 1H), 5.64 (septet, 1H), 3.59 (q, 2H), 2.84 (t, 2H), 2.60 (s, 3H), 2.47 (s, 3H), 1.55 (d, 6H); 412.

### Example 11

### 2-Fluoro-4-[[5-fluoro-4-(2-methyl-3-propan-2-yl-imidazol-4-yl)pyrimidin-2-yl]amino]-N-(2-methylaminoethyl)benzamide

The title compound was prepared by the procedure of Example 1 and on the same scale, using 4-bromo-2-fluoro-N-(2-methylaminoethyl)benzamide (Method 7) and 5-fluoro-4-(3-isopropyl-2-methyl-3*H*-imidazol-4-yl)-pyrimidin-2-ylamine (Method 3). NMR (400.132 MHz, CDCl₃) 8.33 (d, 1H), 8.07 - 8.01 (m, 1H), 7.74 (d of d, 1H), 7.60 (d, 1H), 7.48 (s, 1H), 7.20 (d of d, 1H), 7.14 - 7.07 (m, 1H), 5.56 (septet, 1H), 3.58 (q, 2H), 2.84 (t, 2H), 2.63 (s, 3H), 2.47 (s, 3H), 1.56 (d, 6H); m/z 430.

### Example 12

### N-(2-Dimethylamino-2-methyl-propyl)-4-[[5-fluoro-4-(2-methyl-3-propan-2-yl-imidazol-4-yl)pyrimidin-2-yl]amino]benzamide

4-{[5-Fluoro-4-(1-isopropyl-2-methyl-1*H*-imidazol-5-yl)pyrimidin-2-yl]amino}benzoic acid lithium salt (Method 9) (0.2 g) and HBTU (0.25 g) were dissolved in DMF (5 ml). N,N,2-trimethylpropane-1,2-diamine (0.07 g) was then added followed by DIPEA (0.21 ml) and the reaction mixture was stirred for 16 hrs. The solvent was then removed *in vacuo* and the residue was passed through a SCX-2 column, eluting with MeOH and then with 3.5N NH₃ in MeOH. The solid obtained was recrystallised from a mixture of acetonitrile / ether to give the title compound as a colourless solid (0.05 g). NMR (400.132 MHz, CDCl₃) 8.30 (d, 1H), 7.79 (d, 2H), 7.65 - 7.58 (m, 3H), 7.18 (s, 1H), 6.95 - 6.91 (m, 1H), 5.56 (septet, 1H), 3.35 (d, 2H), 2.62 (s, 3H), 2.24 (s, 6H), 1.54 (d, 6H), 1.08 (s, 6H); m/z 455.

### Example 13

### N-(2-Dimethylamino-2-methyl-propyl)-2-fluoro-4-[[5-fluoro-4-(2-methyl-3-propan-2-yl-imidazol-4-yl)pyrimidin-2-yl]amino]benzamide

The title compound was prepared by the procedure of Example 12 and on the same scale, using 2-fluoro-4-[[5-fluoro-4-(2-methyl-3-propan-2-yl-imidazol-4-yl)pyrimidin-2-yl]amino]benzoic acid lithium salt (Method 11) and N,N,2-trimethylpropane-1,2-diamine. NMR (400.132 MHz, CDCl₃) 8.33 (d, 1H), 8.03 (t, 1H), 7.72 (dd, 1H), 7.60 (d, 1H), 7.45 - 7.37 (m, 1H), 7.27 (s, 1H), 7.20 (dd, 1H), 5.55 (septet, 1H), 3.39 (d, 2H), 2.63 (s, 3H), 2.24 (s, 6H), 1.56 (d, 6H), 1.07 (s, 6H); m/z 472.

### Example 14

### N-(2-Dimethylamino-2-methyl-propyl)-4-[[4-(2-methyl-3-propan-2-yl-imidazol-4-yl)pyrimidin-2-yl]amino]benzamide

The title compound was prepared by the procedure of Example 12 and on the same scale, using 4-{[4-(1-isopropyl-2-methyl-1*H*-imidazol-5-yl)pyrimidin-2-yl]amino}benzoic acid lithium salt (Method 13) and N,N,2-trimethylpropane-1,2-diamine. Purification was by RPHPLC instead of recrystallization. NMR (400.132 MHz, CDCl₃) 8.37 (d, 1H), 7.82 - 7.77 (m, 2H), 7.71 - 7.67 (m, 3H), 7.38 (s, 1H), 7.00 - 6.96 (m, 1H), 6.95 (d, 1H), 5.66 (septet, 1H), 3.36 (d, 2H), 2.59 (s, 3H), 2.24 (s, 6H), 1.53 (d, 6H), 1.07 (s, 6H); m/z 437.

### Example 15

### N-(2-Dimethylamino-2-methyl-propyl)-2-fluoro-4-[[4-(2-methyl-3-propan-2-yl-imidazol-4-yl)pyrimidin-2-yl]amino]benzamide

The title compound was prepared by the procedure of Example 12 and on the same scale, using 2-fluoro-4-[[4-(2-methyl-3-propan-2-yl-imidazol-4-yl)pyrimidin-2-yl]amino]benzoic acid sodium salt (Method 15) and N,N,2-trimethylpropane-1,2-diamine. Purification was by RPHPLC instead of recrystallization. NMR (400.132 MHz, CDCl₃) 8.40 (d, 1H), 8.03 (t, 1H), 7.79 (dd, 1H), 7.45 - 7.37 (m, 2H), 7.32 (s, 1H), 7.24 (dd, 1H), 7.00 (d, 1H), 5.63 (septet, 1H), 3.39 (d, H), 2.60 (s, 3H), 2.24 (s, 6H), 1.56 (d, 6H), 1.07 (s, 6H); m/z 455.

### Example 16

### 4-[[5-Chloro-4-(2-methyl-3-propan-2-yl-imidazol-4-yl)pyrimidin-2-yl]amino]-N-(2-dimethylamino-2-methyl-propyl)benzamide

The title compound was prepared by the procedure of Example 1 and on the same scale, using N-(2-dimethylamino-2-methyl-propyl)-4-iodo-benzamide (Method 16) and 5-chloro-4-(2-methyl-3-propan-2-yl-imidazol-4-yl)pyrimidin-2-amine (Method 5 in WO05/075461). NMR (400.132 MHz, CDCl₃) 8.45 (s, 1H), 7.78 (d, 2H), 7.65 (d, 2H), 7.52 (s, 1H), 7.36 (s, 1H), 6.93 (s, 1H), 4.94 (septet, 1H), 3.35 (d, 2H), 2.60 (s, 3H), 2.24 (s, 6H), 1.49 (d, 6H), 1.07 (s, 6H); m/z 471.

### Example 17

### 4-[[5-Chloro-4-(2-methyl-3-propan-2-yl-imidazol-4-yl)primidin-2-yl]amino]-N-(2-dimethylaminoethyl)benzamide

The title compound was prepared by the procedure of Example 1 and on the same scale, using N-(2-dimethylaminoethyl)-4-iodo-benzamide (Method 4) and 5-chloro-4-(2-methyl-3-propan-2-yl-imidazol-4-yl)pyrimidin-2-amine (Method 5 in WO05/075461). NMR (400.132 MHz, CDCl3) 8.45 (s, 1H), 7.79 (d, 2H), 7.64 (d, 2H), 7.58 (s, 1H), 7.51 (s, 1H), 6.90 (s, 1H), 4.94 (septet, 1H), 3.53 (q, 2H), 2.59 (s, 3H), 2.53 (t, 2H), 2.28 (s, 6H), 1.48 (d, 6H); m/z 443.

### Example 18

### 4-[[5-Chloro-4-(2-methyl-3-propan-2-yl-imidazol-4-yl)pyrimidin-2-yl]amino]-N-(2-dimethylamino-2-methyl-propyl)-2-fluoro-benzamide

The title compound was prepared by the procedure of Example 1 and on the same scale, using 4-bromo-N-(2-dimethylamino-2-methyl-propyl)-2-fluoro-benzamide (Method 17) and 5-chloro-4-(2-methyl-3-propan-2-yl-imidazol-4-yl)pyrimidin-2-amine (Method 5 in WO05/075461). NMR (399.902 MHz, CDCl3) 8.48 (s, 1H), 8.03 (t, 1H), 7.76 (dd, 1H), 7.54 (s, 1H), 7.45 - 7.35 (m, 2H), 7.20 (dd, 1H), 4.95 (septet, 1H), 3.39 (d, 2H), 2.61 (s, 3H), 2.24 (s, 6H), 1.51 (d, 6H), 1.07 (s, 6H); m/z 489.

### Example 19

### 4-[[5-Chloro-4-(2-methyl-3-propan-2-yl-imidazol-4-yl)pyrimidin-2-yl]amino]-2-fluoro-N-[(2S)-2-methylaminopropyl]benzamide

N-Methyl-2,4-dinitro-benzenesulfonamide (in Example 274 in WO01/000206; 288 mg, 1.1 mmol) and triphenylphosphine (315 mg, 1.2 mmol) were added to a stirred solution of 4-[[5-chloro-4-(2-methyl-3-propan-2-yl-imidazol-4-yl)pyrimidin-2-yl]amino]-2-fluoro-N-[(2R)-2-hydroxypropyl]benzamide (Method 19, 446 mg, 1.0 mmol) in THF (15 ml) under an inert atmosphere. DIAD (243 mg, 1.2 mmol) was then added dropwise, the solution stirred for 30 mins before adding additional triphenylphosphine (315 mg, 1.2 mmol) and DIAD (243 mg, 1.2 mmol). After stirring for 30 mins the solvent was evaporated and the residue purified on silica by MPLC (Gilson, 40 g cartridge), eluting with 5% MeOH / DCM to give a mixture of two products. The mixture was dissolved in DCM (10 ml) and n-propylamine (1 ml) was added, after 30 minutes the reaction mixture was evaporated and the residue dissolved in EtOAc. The organic layer was washed with aq. ammonia, dried.(MgSO₄), filtered and concentrated to give a gum. Purification on silica by MPLC (Gilson, 40 g cartridge) eluting with 1% to 10% NH₃-MeOH / DCM followed by crystallisation with ether gave the title compound as a colourless solid (37 mg, 8%). NMR (399.9 MHz) 10.23 (s, 1H), 8.72 (s, 1H), 7.87 (q, 1H), 7.79-7.75 (m, 1H), 7.65 (t, 1H), 7.53-7.50 (m, 1H), 7.28 (s, 1H), 4.86-4.79 (m, 1H), 3.27-3.22 (m, 1H), 3.20-3.16 (m, 1H), 2.68 (q, 1H), 2.50 (s, 3H), 2.30 (s, 3H), 2.00 (bs, 1H), 1.49-1.39 (m, 6H), 0.99 (d, 3H); m/z 461.

### Example 20

### 4-[[5-Chloro-4-(2-methyl-3-propan-2-yl-imidazol-4-yl)pyrimidin-2-yl]amino]-2-fluoro-N-[(2R)-2-methylaminopropyl]benzamide

The title compound was prepared by the procedure of Example 19 and on the same scale, using 4-[[5-chloro-4-(2-methyl-3-propan-2-yl-imidazol-4-yl)pyrimidin-2-yl]amino]-2-fluoro-N-[(2S)-2-hydroxypropyl]benzamide (Method 21). NMR (399.9 MHz) 10.23 (s, 1H), 8.72 (s, 1H), 7.87 (q, 1H), 7.79-7.75 (m, 1H), 7.65 (t, 1H), 7.53-7.50 (m, 1H), 7.28 (s, 1H), 4.86-4.79 (m, 1H), 3.27-3.22 (m, 1H), 3.20-3.16 (m, 1H), 2.68 (q, 1H), 2.50 (s, 3H), 2.30 (s, 3H), 2.00 (bs, 1H), 1.49-1.39 (m, 6H), 0.99 (d, 3H); m/z 461.

### Example 21

### 4-[[5-Cyano-4-(2-methyl-3-propan-2-yl-imidazol-4-yl)pyrrimidin-2-yl]amino]-N-(2-dimethylaminoethyl)-2-fluoro-benzamide

The title compound was prepared by the procedure of Example 1 and on the same scale, using 4-bromo-N-(2-dimethylaminoethyl)-2-fluoro-benzamide (Method 5) and 2-amino-4-(2-methyl-3-propan-2-yl-imidazol-4-yl)pyrimidine-5-carbonitrile (Method 23). NMR (400.132 MHz, CDCl3) 8.70 (s, 1H), 8.09 - 8.05 (m, 2H), 7.85 (s, 1H), 7.74 (d, 1H), 7.30 - 7.27 (m, 1H), 7.25 - 7.22 (m, 1H), 5.23 (septet, 1H), 3.56 (q, 2H), 2.62 (s, 3H), 2.53 (t, 2H), 2.28 (s, 6H), 1.53 (d, 6H); m/z 451.

### Preparation of Starting_materials

### Method 1

### 4-(3-Isopropyl-2-methyl-3H-imidazol-4-yl)-pyrimidin-2-ylamine

(2*E*)-3-(Dimethylamino)-1-(1-isopropyl-2-methyl-1*H*-imidazol-5-yl)prop-2-en-1-one, (Method 24 of WO 03/076436 4.0 g, 18 mmol) and guanidine carbonate (7.2 g, 40 mmol) were pre-mixed in 2-methoxyethanol (80 ml) and heated at reflux for 30 hours. The reaction was allowed to cool before being quenched with water (50 ml). The reaction was then extracted with DCM (2 x 200 ml), dried and solvent was removed *in vacuo* to yield a yellow solid. The solid was dissolved in minimum amount of warm DCM, this was then allowed to cool before the addition of ether. An off white solid precipitated this was filtered and dried. The process was repeated to obtain second crop of product (3.18 g, 81 %). NMR (299.954 MHz, CDCl₃): 8.22 (d, 1H), 7.33 (s, 1H), 6.80 (d, 1H), 5.45 (septet, 1H), 5.10 (s, 2H), 2.56 (s, 3H), 1.54 (d, 6H); m/z 218.

### Method 2

### (2Z)-3-(Dimethylamino)-2-fluoro-1-(1-isopropyl-2-methyl-1H-imidazol-5-yl)prop-2-en-1-one

To a stirred solution of (2*E*)-3-(dimethylamino)-1-(1-isopropyl-2-methyl-1*H-*imidazol-5-yl)prop-2-en-1-one, (Method 24 of WO 03/076436; 5.53 g, 25 mmol) in MeOH (100 ml) at ambient temperature was added in portions over ∼5 mins (1-chloromethyl-4-fluoro-1,4-diazoniabicyclo[2.2.2]octane bis(tetrafluoroborate) (14.16 g, 40 mmol). The temperature was maintained at 25-30°C by slight cooling. After stirring for 90 mins the reaction mixture was cooled in ice/acetone and filtered. The filtrate was evaporated under reduced pressure and the residue was taken into DCM. It was washed with aq. ammonia, brine, dried (Na₂SO₄) and evaporated under reduced pressure. The title compound was isolated by MPLC on silica gel using two separate columns (10% EtOH / EtOAc, then 3.5% EtOH / DCM) as a golden viscose oil, which crystallized on standing over several weeks. Yield = 2.50 g (42%). NMR: 1.40 (d, 6H), 2.38 (s, 3H), 3.05 (s, 6H), 4.70 (septet, 1H), 6.96 (d, 1H), 7.08 (s, 1H); fluorine NMR (376MHz): -166.7 (d); m/z 240.

### Method 3

### 5-Fluoro-4-(3-isopropyl-2-methyl-3H-imidazol-4-yl)-pyrimidin-2-ylamine

(2*Z*)-3-(Dimethylamino)-2-fluoro-1-(1 isopropyl-2-methyl-1*H*-imidazol-5-yl)prop-2-en-1-one (Method 2; 4.0 g, 16.7 mmol) and guanidine carbonate (6.6 g, 37 mmol) were pre-mixed in butanol (80 ml) and heated at reflux for 30 hours. The reaction was allowed to cool before being quenched with water (200 ml) the reaction was then extracted with DCM (2 x 200 ml), dried and solvent was removed *in vacuo* to yield a yellow solid. The solid was dissolved in minimum amount of warm DCM, this was then allowed to cool before the addition of ether. An off white solid precipitated this was filtered and dried. The process was repeated to obtain second crop of product (3.18 g, 81 %). NMR (299.954 MHz, CDCl₃): 8.15 (d, 1H), 7.54 (d, 1H), 7.26 (s, 1H), 5.40 (septet, 1H), 4.88 (s, 2H), 2.59 (s, 3H), 1.56 (d, 6H); m/z 236.

### Method 4

### N-(2-Dimethylaminoethyl)-4-iodo-benzamide

N,N-Dimethylethylenediamine (2 g) was added to a stirred solution of 4-iodobenzoyl chloride (5 g) and TEA (3.9 ml) in DCM (150 ml) under an inert atmosphere. After 1 hr 2M NaOH (50 ml) was added and the mixture extracted with DCM (3 x 100 ml). The combined organics were dried and concentrated *in vacuo* to give the title compound as a brown gum which solidified on standing (4.7 g). NMR (400.132 MHz, CDCl₃) 7.77 (d, 2H), 7.51 (d, 2H), 6.78 (brs, 1H), 3.50 (q, 2H), 2.51 (t, 2H), 2.27 (s, 6H); m/z 319.

### Method 5

### 4-Bromo-N-(2-dimethylaminoethyl)-2-fluoro-benzamide

4-Bromo-2-fluorobenzoic acid (5 g), and HBTU (9.5 g) were dissolved in DMF (150 ml), and then N,N-dimethylethyelenediamine (2.1 g) and DIPEA (10.3 ml) were added. The reaction was stirred for 24 hrs before the removal of the DMF *in vacuo,* the gum was quenched with 2.0M NaOH (50 ml), extracted with DCM (3 x 200 ml), dried and the solvent removed *in vacuo* to yield a viscous gum. Purification by distillation (145°C at 1.2 mmBar) gave the title compound as a yellow gum (5.8 g). NMR (400.132 MHz, CDCl₃) 7.96 (t, 1H), 7.40 (dd, 1H), 7.30 (dd, 1H), 7.20 (brs, 1H), 3.53 (q, 2H), 2.51 (t, 2H), 2.27 (s, 6H); m/z 290.

### Method 6

### 4-Iodo-N-(2-methylaminoethyl)benzamide

tert-Butyl N-(2-aminoethyl)-N-methyl-carbamate (4.16 g) was added to a stirred solution of 4-iodobenzoyl chloride (6.4 g) and TEA (6.2 ml) in DCM (150 ml) under an inert atmosphere. After 20 mins satd. aq. NH₄Cl (50 ml) was added and the mixture was extracted with DCM (3 x 100 ml). The combined organics were dried and concentrated *in vacuo* to give a yellow gum (9.0 g), which was dissolved in acetonitrile (30 ml). 6.0M HCl in propan-2-ol was added and the reaction mixture was stirred overnight. The precipitated solid was filtered and dried to give the title compound as an off-white solid (5.2 g). NMR (400.132 MHz) 9.08 - 8.89 (m, 2H), 7.87 (d, 2H), 7.72 (d, 2H), 3.57 (q, 2H), 3.12 - 3.06 (m, 2H), 2.58 - 2.56 (m, 3H); m/z 305.

### Method 7

### 4-Bromo-2-fluoro-N-(2-methylaminoethyl)benzamide

4-Bromo-2-fluorobenzoic acid (8.2 g), and HBTU (18.4 g) were dissolved in DMF (100 ml), and then tert-butyl N-(2-aminoethyl)-N-methyl-carbamate (6.5 g) and DIPEA (13.3 ml) were added. The reaction was stirred for 24 hrs before adding 2M NaOH (50 ml) then extracting with DCM (3 x 100 ml). The combined organics were dried, filtered and concentrated *in vacuo* to give a yellow gum that was dissolved in MeCN (30 ml). 6M HCl in propan-2-ol was added, the reaction mixture stirred for 24 hrs before filtering and drying to give the title compound as an off-white solid (7.4 g). NMR (400.132 MHz) 9.19 - 9.00 (m, 2H), 8.65 (s, 1H), 7.72 (t, 1H), 7.67 (dd, 1H), 7.53 (dd, 1H), 3.58 (q, 2H), 3.32 (s, 3H), 3.10 - 3.03 (m, 2H); m/z 276.

### Method 8

### Ethyl 4-{[5-fluoro-4-(1-isopropyl-2-methyl-1H-imidazol-5-yl)pyrimidin-2-yl]amino}benzoate

To a solution of 5-fluoro-4-(3-isopropyl-2-methyl-3*H*-imidazol-4-yl)-pyrimidin-2-ylamine (Method 3; 5.32 g) in dioxane (100 ml) was added ethyl 4-iodobenzoate (3.59 g), palladium (II) acetate (305 mg), Xantphos (1.18 g), and caesium carbonate (14.74 g). The mixture was degassed, and purged with nitrogen, then heated at reflux for 3 hrs. The mixture was cooled to ambient temperature, the solids removed by filtration, then the filtrate concentrated in vacuo. Purification on silica using 2-5 % MeOH in DCM as eluent gave the title compound as a yellow solid (2.75 g, 32%). NMR 9.97 (s, 1H), 8.62 (d, 1H), 7.88 (d, 2H), 7.80 (d, 2H), 7.38 (d, 1H), 5.47 - 5.38 (m, 1H), 4.27 (q, 2H), 2.53 (s, 3H), 1.46 (d, 6H), 1.30 (t, 3H); m/z 384.

### Method 9

### 4-{[5-Fluoro-4-(1-isopropyl-2-methyl-1H-imidazol-5-yl)pyrimidin-2-yl]amino}benzoic acid lithium salt

To a stirred solution of ethyl 4-{[5-fluoro-4-(1-isopropyl-2-methyl-1*H*-imidazol-5-yl)pyrimidin-2-yl]amino}benzoate (Method 8; 2.75 g) in EtOH (70 ml) was added a solution of lithium hydroxide (301 mg) in water (15 ml). The mixture was heated under reflux for 18 hrs, then concentrated in vacuo and partitioned between water (300 ml) and EtOAc (300 ml). The aqueous layer was washed with further EtOAc (200 ml) then concentrated in vacuo to yield the title compound as a white solid (2.07 g). NMR 9.56 (s, 1H), 8.53 (d, 1H), 7.80 (d, 2H), 7.53 (d, 2H), 7.36 (d, 1H), 5.56 - 5.46 (m, 1H), 2.51 (s, 3H), 1.43 (d, 6H); m/z 356.

### Method 10

### Ethyl 2-fluoro-4-[[5-fluoro-4-(2-methyl-3-propan-2-yl-imidazol-4-yl)pyrimidin-2-yl]amino]benzoate

The title compound was prepared in similar manner to Method 8 using ethyl 4-bromo-2-fluoro-benzoate in place of ethyl 4-iodobenzoate and purification was achieved by trituration with DCM to give the title compound as a brown solid (3.2 g). NMR (400 MHz) 10.28-10.09 (br s, 1H), 8.68 (d, 1H), 7.87-7.77 (m, 2H), 7.51 (dd, 1H), 7.40 (d, 1H), 5.47-5.34 (m, 1H), 4.29 (q, 2H), 2.55 (s, 3H), 1.48 (d, 6H), 1.30 (t, 3H); m/z 402.

### Method 11

### 2-Fluoro-4-[[5-fluoro-4-(2-methyl-3-propan-2-yl-imidazol-4-yl)pyrimidin-2-yl]amino]benzoic acid lithium salt

The title compound was prepared in similar manner to Method 9 from ethyl 2-fluoro-4-[[5-fluoro-4-(2-methyl-3-propan-2-yl-imidazol-4-yl)pyrimidin-2-yl]amino]benzoate (Method 10) in place of ethyl 4-{[5-fluoro-4-(1-isopropyl-2-methyl-1*H*-imidazol-5-yl)pyrimidin-2-yl]amino}benzoate (Method 8). NMR (400 MHz) 9.68 (br s, 1H), 8.58 (d, 1H), 7.57 (t, 1H), 7.44 (dd, 1H), 7.37 (d, 1H), 7.26 (dd, 1H), 5.54-5.42 (m, 1H), 2.53 (s, 3H), 1.46 (d, 6H); m/z 374.

### Method 12

### Ethyl 4-{[4-(1-isopronyl-2-methyl-1H-imidazol-5-yl)pyrimidin-2-yl]amino}benzoate

To a solution of 4-(3-isopropyl-2-methyl-3*H*-imidazol-4-yl)-pyrimidin-2-ylamine (Method 1; 7.8 g) in dioxane (200 ml) was added ethyl 4-iodobenzoate (9.445 g), palladium (II) acetate (461 mg), Xantphos (1.785 g), and caesium carbonate (22.29 g). The mixture was degassed, and purged with nitrogen, then heated at reflux for 3 hrs. The mixture was cooled to ambient temperature, the solids removed by filtration, and then the filtrate was concentrated in vacuo. Purification on silica using 2-5 % MeOH in DCM as eluent gave the title compound as a yellow solid (3.82 g, 31%). NMR 9.87 (s, 1H), 8.46 (d, 1H), 7.90 - 7.83 (m, 4H), 7.45 (s, 1H), 7.14 (d, 1H), 5.72 - 5.63 (m, 1H), 4.27 (q, 2H), 2.49 (s, 3H), 1.47 (d, 6H), 1.30 (t, 3H); m/z 366.

### Method 13

### 4-{[4-(1-Isopropyl-2-methyl-1H-imidazol-5-yl)pyrimidin-2-yl]amino}benzoic acid lithium salt

The title compound was prepared in similar manner to Method 9 from ethyl 4-{[4-(1-isopropyl-2-methyl-1*H*-imidazol-5-yl)pyrimidin-2-yl]amino}benzoate (Method 12) in place of ethyl 4-{[5-fluoro-4-(1-isopropyl-2-methyl-1*H*-imidazol-5-yl)pyrimidin-2-yl]amino}benzoate (Method 8). NMR (400.132 MHz) 9.51 (s, 1H), 8.40 (d, 1H), 7.85 (d, 2H), 7.61 (d, 2H), 7.43 (s, 1H), 7.05 (d, 1H), 5.78 (m, 1H), 2.50 (s, 3H), 1.45 (d, 6H); m/z 338.

### Method 14

### Methyl 2-fluoro-4-[[4-(2-methyl-3-propan-2-yl-imidazol-4-yl)pyrimidin-2-yl]amino]benzoate

The title compound was prepared in similar manner to Method 9 using methyl 4-bromo-2-fluoro-benzoate in place of ethyl 4-iodobenzoate to give the title compound as a colourless oil (2.27 g). NMR: 10.08 (s, 1H), 8.50 (d, 1H), 7.92-7.76 (m, 2H), 7.54 (d, 1H), 7.46 (s, 1H), 7.19 (d, 1H), 5.72-5.58 (m, 1H), 3.80 (s, 3H), 2.50 (s, 3H under DMSO), 1.48 (d, 6H); m/z 370.

### Method 15

### 2-Fluoro-4-[[4-(2-methyl-3-propan-2-yl-imidazol-4-yl)]pyrimidin-2-yl]amino]benzoic acid sodium salt

Methyl 2-fluoro-4-[[4-(2-methyl-3-propan-2-yl-imidazol-4-yl)pyrimidin-2-yl]amino]benzoate (Method 14; 1.27 g) was dissolved in THF (10 ml) and 1N NaOH was added (3.5 ml). The mixture was heated at reflux for 16 hrs. The reaction mixture was cooled to ambient temperature and the solvent evaporated *in vacuo.* The solid residue was triturated with MeOH then filtered and dried to give a colourless solid (0.8 g). The filtrate was concentrated and triturated with EtOAc, filtered and dried to give a second batch (0.92 g). The two batches were combined to give the title compound as a colourless solid (1.72 g); NMR 400MHz) 9.56 (s, 1H), 8.42 (d, 2H), 7.56 (t, 1H), 7.48 (dd, 1H), 7.44 (s, 1H), 7.24 (dd, 1H), 7.08 (d, 1H), 5.80-5.68 (m, 1H), 2.50 (s, 3H under DMSO), 1.47 (d, 6H); m/z 356.

### Method 16

### N-(2-Dimethylamino-2-methyl-propyl)-4-iodo-benzamide

The title compound was prepared by the procedure of Method 4, using N,N-2-trimethylpropane-1,2-diamine in place of N,N-dimethylethylenediamine and purification was by trituration from MeCN/ DCM to give a colourless solid. NMR (400.132 MHz, CDCl₃) 7.78 (d, 2H), 7.52 (d, 2H), 6.95 (brs, 1H), 3.33 (d, 2H), 2.23 (s, 6H), 1.06 (s, 6H); m/z 347.

### Method 17

### 4-Bromo-N-(2-dimethylamino-2-methyl-propyl)-2-fluoro-benzamide

The title compound was prepared by the procedure of Method 18, using N,N-2-trimethylpropane-1,2-diamine in place of (2R)-1-aminopropan-2-ol. Purification was by MPLC to give the title compound as colourless oil. NMR (399.902 MHz, CDC13) 7.94 (t, 1H), 7.46 (br s, 1H), 7.40 (dd, 1H), 7.31 (dd, 1H), 3.38 (d, 2H), 2.24 (s, 6H), 1.07 (s, 6H); m/z 318.

### Method 18

### 4-Bromo-2-fluoro-N-[(2R)-2-hydroxypropyl]benzamide

4-Bromo-2-fluoro-benzoyl chloride (4.75 g, 20 mmol) was added dropwise to a stirred solution of (2R)-1-aminopropan-2-ol (1.81 g, 24 mmol) and triethylamine (3.03 g, 30 mmol) in DCM (75 ml) at 0-4°C. The solution was warmed to ambient temperature, stirred for 30 mins then washed with 2N HCl, water and sat NaHCO₃. The solvent was evaporated to give a crystalline mass which was triturated with ether / hexane to give the title compound (4.83 g, 88%). NMR (399.9 MHz, CDCl₃) 7.97 (t, 1H), 7.43-7.41 (m, 1H), 7.35-7.31 (m, 1H), 7.05 (s, 1H), 4.08-4.03 (m, 1H), 3.70-3.64 (m, 1H), 3.38-3.31 (m, 1H), 2.30 (d, 1H), 1.26 (d, 3H); m/z 277.

### Method 19

### 4-[[5-Chloro-4-(2-methyl-3-propan-2-yl-imidazol-4-yl)pyrimidin-2-yl]amino]-2-fluoro-N-[(2R)-2-hydroxypropyl]benzamide

The title compound was prepared by the procedure of Example 1, using 4-bromo-2-fluoro-N-[(2R)-2-hydroxypropyl]benzamide (Method 18) and 5-chloro-4-(2-methyl-3-propan-2-yl-imidazol-4-yl)pyrimidin-2-amine (Method 5 in WO05/075461). NMR (399.9 MHz) 10.23 (s, 1H), 8.72 (s, 1H), 7.83 (q, 1H), 7.80-7.76 (m, 1H), 7.67 (t, 1H), 7.52-7.49 (m, 1H), 7.28 (s, 1H), 4.87-4.80 (m, 1H), 4.76-4.75 (m, 1H), 3.81-3.75 (m, 1H), 3.28-3.23 (m, 1H), 3.21-3.18 (m, 1H), 2.50 (s, 3H), 1.43 (d, 6H), 1.08 (d, 3H); m/z 448.

### Method 20

### 4-Bromo-2-fluoro-N-[(2S)-2-hydroxypropyl]benzamide

The title compound was prepared by the procedure of Method 18, using (2S)-1-aminopropan-2-ol in place of (2R)-1-aminopropan-2-ol. NMR (399.9 MHz; CDCl₃) 7.97 (t, 1H), 7.43-7.41 (m, 1H), 7.35-7.31 (m, 1H), 7.05 (s, 1H), 4.08-4.03 (m, 1H), 3.70-3.64 (m, 1H), 3.38-3.31 (m, 1H), 2.30 (d, 1H), 1.26 (d, 3H); m/z 277.

### Method 21

### 4-[[5-Chloro-4-(2-methyl-3-propan-2-yl-imidazol-4-yl)pyrimidin-2-yl]amino]-2-fluoro-N-[(2S)-2-hydroxypropyl]benzamide

The title compound was prepared by the procedure of Example 1, using 4-bromo-2-fluoro-N-[(2S)-2-hydroxypropyl]benzamide (Method 20) and 5-chloro-4-(2-methyl-3-propan-2-yl-imidazol-4-yl)pyrimidin-2-amine (Method 5 in WO05/075461). NMR (399.9 MHz) 10.23 (s, 1H), 8.72 (s, 1H), 7.83 (q, 1H), 7.80-7.76 (m, 1H), 7.67 (t, 1H), 7.52-7.49 (m, 1H), 7.28 (s, 1H), 4.87-4.80 (m, 1H), 4.76-4.75 (m, 1H), 3.81-3.75 (m, 1H), 3.28-3.23 (m, 1H), 3.21-3.18 (m, 1H), 2.50 (s, 3H), 1.43 (d, 6H), 1.08 (d, 3H); m/z 448.

### Method 22

### 5-Iodo-4-(2-methyl-3-propan-2-yl-imidazol-4-yl)pyrimidin-2-amine

A solution of 4-(3-isopropyl-2-methyl-3*H*-imidazol-4-yl)-pyrimidin-2-ylamine (Method 1, 3 g, 13.8 mmol) and N-iodosuccinimide (3.3 g, 14.5 mmol) in acetic acid (35 ml) was heated at 50°C for 16 hrs. The reaction was evaporated, quenched with aq. sodium thiosulphate until colourless and then 2.0 M NaOH (50 ml) was added. The aqueous layer was extracted with DCM (3 x 200 ml), the combined organics were dried (MgSO₄) and the solvent evaporated to give a colourless solid. The solid was added to acetonitrile, heated to 60°C and then allowed to cool before being filtered, the process was repeated on the filtrate to give the title compound as a colourless solid (3.5 g, 74%). NMR (400.132 MHz, CDCl3) 8.62 (s, 1H), 7.34 (s, 1H), 5.08 (br s, 2H), 4.57 (septet, 1H), 2.55 (s, 3H), 1.48 (d, 6H); m/z 344.

### Method 23

### 2-Amino-4-(2-methyl-3-propan-2-yl-imidazol-4-yl)pyrimidine-5-carbonitrile

A solution of 5-iodo-4-(2-methyl-3-propan-2-yl-imidazol-4-yl)pyrimidin-2-amine (Method 22, 2.2 g, 6.4 mmol) and CuCN (2.2 g, 12.8 mmol) in pyridine (60 ml) was heated under reflux for 2 days. The solvent was evaporated, the residue was dissolved in DMF and loaded onto a SCX column (50 g), washing with DMF and MeOH then eluting with 7M ammonia/ MeOH. The residue obtained was dissolved in DCM, then 2.0 M NaOH (100 ml) was added and the aqueous layer extracted with additional DCM (3 x 200 ml). The combined organics were dried and the solvent evaporated to give a yellow solid, which was dissolved in the minimum amount of hot acetonitrile, cooled and filtered. The process was repeated with the filtrate to give the title compound as an off-white solid (0.81 g, 52%). NMR (400.132 MHz, CDC13) 8.54 (s, 1H), 7.79 (s, 1H), 5.60 (brs, 2H), 5.11 (septet, 1H), 2.59 (s, 3H), 1.55 (d, 6H); m/z 243.

### Example 22

The following illustrate representative pharmaceutical dosage forms containing the compound of formula (I), or a pharmaceutically acceptable salt thereof (hereafter compound X), for therapeutic or prophylactic use in humans:-

| **(a): Tablet I** | **mg/tablet** |
|---|---|
| Compound X | 100 |
| Lactose Ph.Eur | 182.75 |
| Croscarmellose sodium | 12.0 |
| Maize starch paste (5% w/v paste) | 2.25 |
| Magnesium stearate | 3.0 |
| | |

| **(b): Tablet II** | **mg/tablet** |
|---|---|
| Compound X | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |
| | |

| **(c): Tablet III** | **mg/tablet** |
|---|---|
| Compound X | 1.0 |
| Lactose Ph.Eur | 93.25 |
| Croscarmellose sodium | 4.0 |
| Maize starch paste (5% w/v paste) | 0.75 |
| Magnesium stearate | 1.0 |
| | |

| **(d): Capsule** | **mg/capsule** |
|---|---|
| Compound X | 10 |
| Lactose Ph.Eur | 488.5 |
| Magnesium stearate | 1.5 |

| **(e): Injection I** | **(50 mg/ml)** |
|---|---|
| Compound X | 5.0% w/v |
| 1M Sodium hydroxide solution | 15.0% v/v |
| 0.1M Hydrochloric acid | (to adjust pH to 7.6) |
| Polyethylene glycol 400 | 4.5% w/v |
| Water for injection | to 100% |
| | |

| **(f): Injection II** | **10 mg/ml** |
|---|---|
| Compound X | 1.0% w/v |
| Sodium phosphate BP | 3.6% w/v |
| 0.1M Sodium hydroxide solution | 15.0% v/v |
| Water for injection | to 100% |
| | |

| **(g): Injection III** | **(1 mg/ml,buffered to pH6)** |
|---|---|
| Compound X | 0.1% w/v |
| Sodium phosphate BP | 2.26% w/v |
| Citric acid | 0.38% w/v |
| Polyethylene glycol 400 | 3.5% w/v |
| Water for injection | to 100% |

| | |
|---|---|
| Note The above formulations may be obtained by conventional procedures well known in the pharmaceutical art. The tablets (a)-(c) may be enteric coated by conventional means, for example to provide a coating of cellulose acetate phthalate. | |

## Claims

1. A compound of formula **(I):** 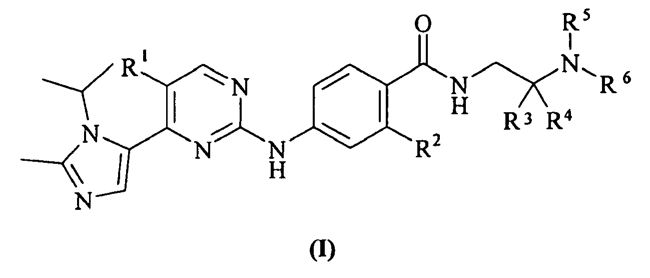 wherein:
**R¹** is hydrogen, halo or cyano;
**R²** is hydrogen or halo;
**R³, R⁴, R⁵** and **R⁶** are independently selected from hydrogen and methyl optionally substituted by hydroxy;
or a pharmaceutically acceptable salt thereof.

2. A compound of formula **(I),** or a pharmaceutically acceptable salt thereof, as claimed in claim 1 wherein R¹ is hydrogen, fluoro, chloro or cyano.

3. A compound of formula **(I),** or a pharmaceutically acceptable salt thereof, as claimed in either claim 1 or claim 2 wherein R² is hydrogen or fluoro.

4. A compound of formula **(I),** or a pharmaceutically acceptable salt thereof, as claimed in any one of claims 1-3 wherein R³ is hydrogen or methyl.

5. A compound of formula **(I),** or a pharmaceutically acceptable salt thereof, as claimed in any one of claims 1-4 wherein R⁴ is hydrogen or methyl.

6. A compound of formula **(I),** or a pharmaceutically acceptable salt thereof, as claimed in any one of claims 1-5 wherein R⁵ is methyl.

7. A compound of formula **(I),** or a pharmaceutically acceptable salt thereof, as claimed in any one of claims 1-6 wherein R⁶ is hydrogen or methyl.

8. A compound of formula **(I)** as claimed in claim 1:
wherein:
R¹ is hydrogen, fluoro, chloro or cyano;
R² is hydrogen or fluoro;
R³ and R⁴ are hydrogen or methyl;
one of R⁵ and R⁶ is methyl and the other is hydrogen or methyl;
or a pharmaceutically acceptable salt thereof.

9. A compound of formula **(I)** as claimed in claim 1:
selected from:
N-(2-dimethylaminoethyl)-4-[[4-(2-methyl-3-propan-2-yl-imidazol-4-yl)pyrimidin-2-yl]amino]benzamide;
N-(2-dimethylaminoethyl)-2-fluoro-4-[[4-(2-methyl-3-propan-2-yl-imidazol-4-yl)pyrimidin-2-yl]amino]benzamide;
N-(2-dimethylaminoethyl)-4-[[5-fluoro-4-(2-methyl-3-propan-2-yl-imidazol-4-yl)pyrimidin-2-yl]amino]benzamide;
N-(2-dimethylaminoethyl)-2-fluoro-4-[[5-fluoro-4-(2-methyl-3-propan-2-yl-imidazol-4-yl)pyrimidin-2-yl]amino]benzamide;
4-[[5-chloro-4-(2-methyl-3-propan-2-yl-imidazol-4-yl)pyrimidin-2-yl]amino]-N-(2-dimethylaminoethyl)-2-fluoro-benzamide;
N-(2-methylaminoethyl)-4-[[4-(2-methyl-3-propan-2-yl-imidazol-4-yl)pyrimidin-2-yl]amino]benzamide;
4-[[5-fluoro-4-(2-methyl-3-propan-2-yl-imidazol-4-yl)pyrimidin-2-yl]amino]-N-(2-methylaminoethyl)benzamide;
4-[[5-chloro-4-(2-methyl-3-propan-2-yl-imidazol-4-yl)pyrimidin-2-yl]amino]-N-(2-methylaminoethyl)benzamide;
4-[[5-chloro-4-(2-methyl-3-propan-2-yl-imidazol-4-yl)pyrimidin-2-yl]amino]-2-fluoro-N-(2-methylaminoethyl)benzamide;
2-fluoro-N-(2-methylaminoethyl)-4-[[4-(2-methyl-3-propan-2-yl-imidazol-4-yl)pyrimidin-2-yl]amino]benzamide;
2-fluoro-4-[[5-fluoro-4-(2-methyl-3-propan-2-yl-imidazol-4-yl)pyrimidin-2-yl]amino]-N-(2-methylaminoethyl)benzamide;
N-(2-dimethylamino-2-methyl-propyl)-4-[[5-fluoro-4-(2-methyl-3-propan-2-yl-imidazol-4-yl)pyrimidin-2-yl]amino]benzamide;
N-(2-dimethylamino-2-methyl-propyl)-2-fluoro-4-[[5-fluoro-4-(2-methyl-3-propan-2-yl-imidazol-4-yl)pyrimidin-2-yl]amino]benzamide;
N-(2-dimethylamino-2-methyl-propyl)-4-[[4-(2-methyl-3-propan-2-yl-imidazol-4-yl)pyrimidin-2-yl]amino]benzamide;
N-(2-dimethylamino-2-methyl-propyl)-2-fluoro-4-[[4-(2-methyl-3-propan-2-yl-imidazol-4-yl)pyrimidin-2-yl]amino]benzamide;
4-[[5-chloro-4-(2-methyl-3-propan-2-yl-imidazol-4-yl)pyrimidin-2-yl]amino]-N-(2-dimethylamino-2-methyl-propyl)benzamide;
4-[[5-chloro-4-(2-methyl-3-propan-2-yl-imidazol-4-yl)pyrimidin-2-yl]amino]-N-(2-dimethylaminoethyl)benzamide;
4-[[5-chloro-4-(2-methyl-3-propan-2-yl-imidazol-4-yl)pyrimidin-2-yl]amino]-N-(2-dimethylamino-2-methyl-propyl)-2-fluoro-benzamide;
4-[[5-chloro-4-(2-methyl-3-propan-2-yl-imidazol-4-yl)pyrimidin-2-yl]amino]-2-fluoro-N-[(2S)-2-methylaminopropyl]benzamide;
4-[[5-chloro-4-(2-methyl-3-propan-2-yl-imidazol-4-yl)pyrimidin-2-yl]amino]-2-fluoro-N-[(2R)-2-methylaminopropyl]benzamide; and
4-[[5-cyano-4-(2-methyl-3-propan-2-yl-imidazol-4-yl)pyrimidin-2-yl]amino]-N-(2-dimethylaminoethyl)-2-fluoro-benzamide;
or a pharmaceutically acceptable salt thereof.

10. A pharmaceutical composition which comprises a compound of the formula **(I),** or a pharmaceutically acceptable salt thereof, as claimed in any one of claims 1-9, and a pharmaceutically-acceptable diluent or carrier.

11. A compound of the formula **(I),** or a pharmaceutically acceptable salt thereof, as claimed in any one of claims 1-9, for use as a medicament.

12. The use of a compound of the formula **(I),** or a pharmaceutically acceptable salt thereof, as claimed in any one of claims 1-9, in the manufacture of a medicament for the treatment of cancer.

13. The use of a compound of the formula **(I),** or a pharmaceutically acceptable salt thereof, as claimed in any one of claims 1-9, in the manufacture of a medicament for the treatment of leukaemia or lymphoid malignancies or cancer of the breast, lung, colon, rectum, stomach, liver, kidney, prostate, bladder, pancreas, vulva, skin or ovary.

14. The use of a compound of the formula **(I),** or a pharmaceutically acceptable salt thereof, as claimed in any one of claims 1-9, in the manufacture of a medicament for the treatment of cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

15. A pharmaceutical product comprising a compound of the formula **(I)** as claimed in any of claims 1-9, and an additional anti-tumour substance for the conjoint treatment of cancer.

## Patentansprüche

1. Verbindung der Formel (I): 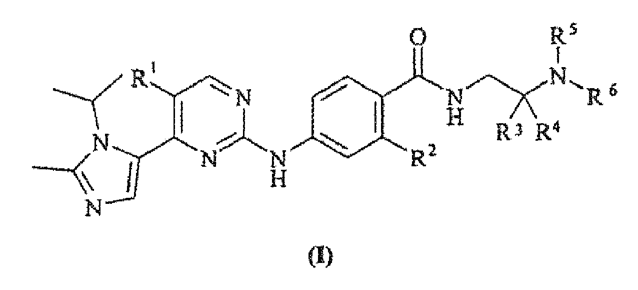 worin:
R¹ für Wasserstoff, Halogen oder Cyano steht;
R² für Wasserstoff oder Halogen steht;
R³, R⁴, R⁵ und R⁶ unabhängig voneinander unter Wasserstoff und gegebenenfalls durch Hydroxy substituiertem Methyl ausgewählt sind;
oder ein pharmazeutisch annehmbares Salz davon.

2. Verbindung der Formel (I) oder ein pharmazeutisch annehmbares Salz davon nach Anspruch 1, worin R¹ für Wasserstoff, Fluor, Chlor oder Cyano steht.

3. Verbindung der Formel (I) oder ein pharmazeutisch annehmbares Salz davon nach Anspruch 1 oder Anspruch 2, worin R² für Wasserstoff oder Fluor steht.

4. Verbindung der Formel (I) oder ein pharmazeutisch annehmbares Salz davon nach einem der Ansprüche 1-3, worin R³ für Wasserstoff oder Methyl steht.

5. Verbindung der Formel (I) oder ein pharmazeutisch annehmbares Salz davon nach einem der Ansprüche 1-4, worin R⁴ für Wasserstoff oder Methyl steht.

6. Verbindung der Formel (I) oder ein pharmazeutisch annehmbares Salz davon nach einem der Ansprüche 1-5, worin R⁵ für Methyl steht.

7. Verbindung der Formel (I) oder ein pharmazeutisch annehmbares Salz davon nach einem der Ansprüche 1-6, worin R⁶ für Wasserstoff oder Methyl steht.

8. Verbindung der Formel (I) nach Anspruch 1,
worin:
R¹ für Wasserstoff, Fluor, Chlor oder Cyano steht;
R² für Wasserstoff oder Fluor steht;
R³ und R⁴ für Wasserstoff oder Methyl stehen;
eine der Gruppen R⁵ und R⁶ für Methyl steht und die andere für Wasserstoff oder Methyl steht;
oder ein pharmazeutisch annehmbares Salz davon.

9. Verbindung der Formel (I) nach Anspruch 1, ausgewählt unter:
N-(2-Dimethylaminoethyl)-4-[[4-(2-methyl-3-propan-2-ylimidazol-4-yl)pyrimidin-2-yl]amino]benzamid;
N-(2-Dimethylaminoethyl)-2-fluor-4-[[4-(2-methyl-3-propan-2-ylimidazol-4-yl)pyrimidin-2-yl]amino]-benzamid;
N-(2-Dimethylaminoethyl)-4-[[5-fluor-4-(2-methyl-3-propan-2-ylimidazol-4-yl)pyrimidin-2-yl]amino]-benzamid;
N-(2-Dimethylaminoethyl)-2-fluor-4-[[5-fluor-4-(2-methyl-3-propan-2-ylimidazol-4-yl)pyrimidin-2-yl]-amino]benzamid;
4-[[5-Chlor-4-(2-methyl-3-propan-2-ylimidazol-4-yl)pyrimidin-2-yl]amino]-N-(2-dimethylaminoethyl)-2-fluorbenzamid;
N-(2-Methylaminoethyl)-4-[[4-(2-methyl-3-propan-2-ylimidazol-4-yl)pyrimidin-2-yl]amino]benzamid;
4-[[5-Fluor-4-(2-methyl-3-propan-2-ylimidazol-4-yl)pyrimidin-2-yl]amino]-N-(2-methylaminoethyl)-benzamid;
4-[[5-Chlor-4-(2-methyl-3-propan-2-ylimidazol-4-yl)pyrimidin-2-yl]amino]-N-(2-methylaminoethyl)-benzamid;
4-[[5-Chlor-4-(2-methyl-3-propan-2-ylimidazol-4-yl)pyrimidin-2-yl]amino]-2-fluor-N-(2-methylaminoethyl)benzamid;
2-Fluor-N-(2-methylaminoethyl)-4-[[4-(2-methyl-3-propan-2-ylimidazol-4-yl)pyrimidin-2-yl]amino]-benzamid;
2-Fluor-4-[[5-fluor-4-(2-methyl-3-propan-2-ylimidazol-4-yl)pyrimidin-2-yl]amino]-N-(2-methylaminoethyl)benzamid;
N-(2-Dimethylamino-2-methylpropyl)-4-[[5-fluor-4-(2-methyl-3-propan-2-ylimidazol-4-yl)pyrimidin-2-yl]amino]benzamid;
N-(2-Dimethylamino-2-methylpropyl)-2-fluor-4-[[5-fluor-4-(2-methyl-3-propan-2-ylimidazol-4-yl)-pyrimidin-2-yl]amino]benzamid;
N-(2-Dimethylamino-2-methylpropyl)-4-[[4-(2-methyl-3-propan-2-ylimidazol-4-yl)pyrimidin-2-yl]-amino]benzamid;
N-(2-Dimethylamino-2-methylpropyl)-2-fluor-4-[[4-(2-methyl-3-propan-2-ylimidazol-4-yl)pyrimidin-2-yl]amino]benzamid;
4-[[5-Chlor-4-(2-methyl-3-propan-2-ylimidazol-4-yl)pyrimidin-2-yl]amino]-N-(2-dimethylamino-2-methylpropyl)benzamid;
4-[[5-Chlor-4-(2-methyl-3-propan-2-ylimidazol-4-yl)pyrimidin-2-yl]amino]-N-(2-dimethylaminoethyl)-benzamid;
4-[[5-Chlor-4-(2-methyl-3-propan-2-ylimidazol-4-yl)pyrimidin-2-yl]amino]-N-(2-dimethylamino-2-methylpropyl)-2-fluorbenzamid;
4-[[5-Chlor-4-(2-methyl-3-propan-2-ylimidazol-4-yl)pyrimidin-2-yl]amino]-2-fluor-N-[(2S)-2-methylaminopropyl]benzamid;
4-[[5-Chlor-4-(2-methyl-3-propan-2-ylimidazol-4-yl)pyrimidin-2-yl]amino]-2-fluor-N-[(2R)-2-methylaminopropyl]benzamid und
4-[[5-Cyano-4-(2-methyl-3-propan-2-ylimidazol-4-yl)pyrimidin-2-yl]amino]-N-(2-dimethylaminoethyl)-2-fluorbenzamid;
oder ein pharmazeutisch annehmbares Salz davon.

10. Pharmazeutische Zusammensetzung, die eine Verbindung der Formel (I) oder ein pharmazeutisch annehmbares Salz davon nach einem der Ansprüche 1-9 und ein pharmazeutisch annehmbares Verdünnungsmittel oder einen pharmazeutisch annehmbaren Träger enthält.

11. Verbindung der Formel (I) oder ein pharmazeutisch annehmbares Salz davon nach einem der Ansprüche 1-9 zur Verwendung als Arzneimittel.

12. Verwendung einer Verbindung der Formel (I) oder eines pharmazeutisch annehmbaren Salzes davon nach einem der Ansprüche 1-9 bei der Herstellung eines Arzneimittels zur Behandlung von Krebs.

13. Verwendung einer Verbindung der Formel (I) oder eines pharmazeutisch annehmbaren Salzes davon nach einem der Ansprüche 1-9 bei der Herstellung eines Arzneimittels zur Behandlung von Leukämie oder lymphoiden Malignomen oder Brust-, Lungen-, Kolon-, Rektum-, Magen-, Leber-, Nieren-, Prostata-, Blasen-, Bauchspeicheldrüsen-, Vulva-, Haut- oder Eierstockkrebs.

14. Verwendung einer Verbindung der Formel (I) oder eines pharmazeutisch annehmbaren Salzes davon nach einem der Ansprüche 1-9 bei der Herstellung eines Arzneimittels zur Behandlung von Krebs, fibroproliferativen und differentiativen Erkrankungen, Psoriasis, rheumatoider Arthritis, Kaposi-Sarkom, Hämangiom, akuter oder chronischer Nephropathie, Atherom, Atherosklerose, arterieller Restenose, Autoimmunerkrankungen, akuten oder chronischen Entzündungen, Knochenerkrankungen oder Augenerkrankungen mit Netzhautgefäßproliferation.

15. Pharmazeutisches Produkt, das eine Verbindung der Formel (I) nach einem der Ansprüche 1-9 und eine zusätzliche Antitumorsubstanz enthält, zur Kombinationsbehandlung von Krebs.

## Revendications

1. Composé de formule (I) : 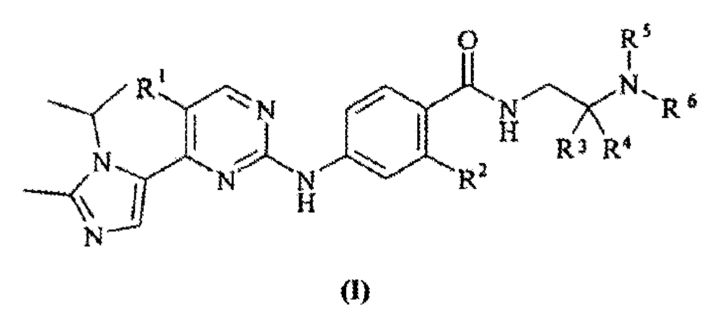 dans laquelle :
R¹ est un hydrogène, halo ou cyano ;
R² est un hydrogène ou halo ;
R³, R⁴, R⁵ et R⁶ sont indépendamment choisis parmi un hydrogène et un méthyle facultativement substitué par un hydroxy ;
ou sel pharmaceutiquement acceptable de celui-ci.

2. Composé de formule (I), ou sel pharmaceutiquement acceptable de celui-ci, selon la revendication 1 dans lequel R¹ est un hydrogène, fluoro, chloro ou cyano.

3. Composé de formule (I), ou sel pharmaceutiquement acceptable de celui-ci, selon l'une ou l'autre de la revendication 1 ou de la revendication 2 dans lequel R² est un hydrogène ou fluoro.

4. Composé de formule (I), ou sel pharmaceutiquement acceptable de celui-ci, selon l'une quelconque des revendications 1-3 dans lequel R³ est un hydrogène ou méthyle.

5. Composé de formule (I), ou sel pharmaceutiquement acceptable de celui-ci, selon l'une quelconque des revendications 1-4 dans lequel R⁴ est un hydrogène ou méthyle.

6. Composé de formule (I), ou sel pharmaceutiquement acceptable de celui-ci, selon l'une quelconque des revendications 1-5 dans lequel R⁵ est un méthyle.

7. Composé de formule (I), ou sel pharmaceutiquement acceptable de celui-ci, selon l'une quelconque des revendications 1-6 dans lequel R⁶ est un hydrogène ou méthyle.

8. Composé de formule (I) selon la revendication 1 :
dans lequel :
R¹ est un hydrogène, fluoro, chloro ou cyano ;
R² est un hydrogène ou fluoro ;
R³ et R⁴ sont un hydrogène ou méthyle ;
l'un de R⁵ et R⁶ est un méthyle et l'autre est un hydrogène ou méthyle ;
ou sel pharmaceutiquement acceptable de celui-ci.

9. Composé de formule (I) selon la revendication 1 :
choisi parmi :
le *N*-(2-diméthylaminoéthyl)-4-[[4-(2-méthyl-3-propan-2-ylimidazol-4-yl)pyrimidin-2-yl]amino]benzamide ;
le *N*-(2-diméthylaminoéthyl)-2-fluoro-4-[[4-(2-méthyl-3-propan-2-ylimidazol-4-yl)pyrimidin-2-yl]amino]benzamide ;
le *N*-(2-diméthylaminoéthyl)-4-[[5-fluoro-4-(2-méthyl-3-propan-2-ylimidazol-4-yl)pyrimidin-2-yl]amino]benzamide ;
le *N*-(2-diméthylaminoéthyl)-2-fluoro-4-[[5-fluoro-4-(2-méthyl-3-propan-2-ylimidazol-4-yl)pyrimidin-2-yl]amino]benzamide ;
le 4-[[5-chloro-4-(2-méthyl-3-propan-2-ylimidazol-4-yl)pyrimidin-2-yl]amino]-*N*-(2-diméthylaminoéthyl)-2-fluorobenzamide ;
le *N*-(2-méthylaminoéthyl)-4-[[4-(2-méthyl-3-propan-2-ylimidazol-4-yl)pyrimidin-2-yl]amino]benzamide ;
le 4-[[5-fluoro-4-(2-méthyl-3-propan-2-ylimidazol-4-yl)pyrimidin-2-yl]amino]-*N*-(2-méthylaminoéthyl)benzamide ;
le 4-[[5-chloro-4-(2-méthyl-3-propan-2-ylimidazol-4-yl)pyrimidin-2-yl]amino]-*N*-(2-méthylaminoéthyl)benzamide ;
le 4-[[5-chloro-4-(2-méthyl-3-propan-2-ylimidazol-4-yl)pyrimidin-2-yl]amino]-2-fluoro-*N*-(2-méthylaminoéthyl)-benzamide ;
le 2-fluoro-*N*-(2-méthylaminoéthyl)-4-[[4-(2-méthyl-3-propan-2-ylimidazol-4-yl)pyrimidin-2-yl]amino]benzamide ;
le 2-fluoro-4-[[5-fluoro-4-(2-méthyl-3-propan-2-ylimidazol-4-yl)pyrimidin-2-yl]amino]-*N*-(2-méthylamino-éthyl)benzamide ;
le *N*-(2-diméthylamino-2-méthylpropyl)-4-[[5-fluoro-4-(2-méthyl-3-propan-2-ylimidazol-4-yl)pyrimidin-2-yl]amino]benzamide ;
le *N*-(2-diméthylamino-2-méthylpropyl)-2-fluoro-4-[[5-fluoro-4-(2-méthyl-3-propan-2-ylimidazol-4-yl)pyrimidin-2-yl]amino]benzamide ;
le *N*-(2-diméthylamino-2-méthylpropyl)-4-[[4-(2-méthyl-3-propan-2-ylimidazol-4-yl)pyrimidin-2-yl]amino]benzamide ;
le *N*-(2-diméthylamino-2-méthylpropyl)-2-fluoro-4-[[4-(2-méthyl-3-propan-2-ylimidazol-4-yl)pyrimidin-2-yl]amino]benzamide ;
le 4-[[5-chloro-4-(2-méthyl-3-propan-2-ylimidazol-4-yl)pyrimidin-2-yl]amino]-*N*-(2-diméthylamino-2-méthylpropyl)benzamide ;
le 4-[[5-chloro-4-(2-méthyl-3-propan-2-ylimidazol-4-yl)pyrimidin-2-yl]amino]-*N*-(2-diméthylaminoéthyl)-benzamide ;
le 4-[[5-chloro-4-(2-méthyl-3-propan-2-ylimidazol-4-yl)pyrimidin-2-yl]amino]-*N*-(2-diméthylamino-2-méthylpropyl)-2-fluorobenzamide ;
le 4-[[5-chloro-4-(2-méthyl-3-propan-2-ylimidazol-4-yl)pyrimidin-2-yl]amino]-2-fluoro-*N*-[(2S)-2-méthylaminopropyl]benzamide ;
le 4-[[5-chloro-4-(2-méthyl-3-propan-2-ylimidazol-4-yl)pyrimidin-2-yl]amino]-2-fluoro-*N*-[(2R)-2-méthylaminopropyl]benzamide ; et
le 4-[[5-cyano-4-(2-méthyl-3-propan-2-ylimidazol-4-yl)pyrimidin-2-yl]amino]-*N*-(2-diméthylaminoéthyl)-2-fluorobenzamide ;
ou sel pharmaceutiquement acceptable de celui-ci.

10. Composition pharmaceutique qui comprend un composé représenté par la formule (I), ou un sel pharmaceutiquement acceptable de celui-ci, selon l'une quelconque des revendications 1-9, et un diluant ou véhicule pharmaceutiquement acceptable.

11. Composé représenté par la formule (I), ou sel pharmaceutiquement acceptable de celui-ci, selon l'une quelconque des revendications 1-9, destiné à être utilisé comme médicament.

12. Utilisation d'un composé représenté par la formule (I), ou d'un sel pharmaceutiquement acceptable de celui-ci, selon l'une quelconque des revendications 1-9, dans la fabrication d'un médicament pour le traitement d'un cancer.

13. Utilisation d'un composé représenté par la formule (I), ou d'un sel pharmaceutiquement acceptable de celui-ci, selon l'une quelconque des revendications 1-9, dans la fabrication d'un médicament pour le traitement d'une leucémie ou de tumeurs malignes lymphoïdes ou d'un cancer du sein, du poumon, du côlon, du rectum, de l'estomac, du foie, du rein, de la prostate, de la vessie, du pancréas, de la vulve, de la peau ou de l'ovaire.

14. Utilisation d'un composé représenté par la formule (I), ou d'un sel pharmaceutiquement acceptable de celui-ci, selon l'une quelconque des revendications 1-9, dans la fabrication d'un médicament pour le traitement d'un cancer, de troubles de prolifération fibreuse et de la différenciation, du psoriasis, de la polyarthrite rhumatoïde, du sarcome de Kaposi, d'un angiome des vaisseaux sanguins, de néphropathies aiguës et chroniques, d'un athérome, de l'athérosclérose, d'une resténose artérielle, de maladies autoimmunes, d'une inflammation aiguë ou chronique, de maladies osseuses et de maladies oculaires avec prolifération de vaisseaux rétiniens.

15. Produit pharmaceutique comprenant un composé représenté par la formule (I) selon l'une quelconque des revendications 1-9 et une substance antitumorale supplémentaire pour le traitement conjoint d'un cancer.
